# EUROPEAN PATENT APPLICATION

(11) **EP 4 067 371 A1**
(43) Date of publication of application: **05.10.2022**
(21) Application number: 21166438.8
(22) Date of filing: 31.03.2021
(51) Int. Cl.: C07K 14/435

(54) **T-CELL RECEPTORS SPECIFIC FOR HEPATITIS C VIRUS PEPTIDES**

(71) Applicant: Max-Delbrück-Centrum für Molekulare Medizin in der Helmholtz-Gemeinschaft, 13125 Berlin (DE); Charité - Universitätsmedizin Berlin, 10117 Berlin (DE)
(72) Inventor: BLANKENSTEIN, Thomas, 12209 Berlin (DE); THIEDE, Katerina, 12049 Berlin (DE); WILLIMSKY, Gerald, 10405 Berlin (DE)
(74) Representative: Kuttenkeuler, David

(57) **Abstract**

The invention is based on antigen binding proteins (ABPs) such as T-cell receptors (TCR) expressed on T-cells, which have a specificity to bind to MHC presented Hepatitis C Virus peptides. Provided are isolated ABPs as well as genetic constructs expressing the ABPs, recombinant host cells harboring the ABP of the invention and methods for producing such ABPs and host cells. Moreover, provided are medical applications involving the TCR of the invention, for example in context of an adoptive T-cell therapy.

## Description

### FIELD OF THE INVENTION

The invention is based on antigen binding proteins (ABPs) such as T-cell receptors (TCR) expressed on T-cells, which have a specificity to bind to MHC presented Hepatitis C Virus peptides. Provided are isolated ABPs as well as genetic constructs expressing the ABPs, recombinant host cells harboring the ABP of the invention and methods for producing such ABPs and host cells. Moreover, provided are medical applications involving the TCR of the invention, for example in context of an adoptive T-cell therapy.

### DESCRIPTION

Human T-cells are lymphocytes that originate from bone marrow progenitor cells, and are matured in the thymus. Several T-cell subtypes are distinguished, which are categorized into CD4+ T helper cells and CD8+ T killer cells. The different types of CD₄+ T-cells generally mediate immune functions: For example, Th1 cells are connected to immune responses against intracellular bacteria, fungi and protozoa and secrete INF-gamma and can enhance the microbial activity of macrophages. Th2 cells on the other hand play a role in the humoral immune response. By cytokine secretion and surface proteins interactions, Th2 play an important role in B-cell stimulation to secrete antibodies. CD8+ T-cells are specialized in recognizing infected or mutated cells. Upon recognition of a diseased cell, they release a mixture of perforins, granzymes and chemokines that ultimately result in apoptosis of the infected cells. (Nurieva R., Chung Y., Cellular & Molecular Immunology, 2010, 7, 190-197).

T-cell activation depends amongst others on the interaction of their unique T-cell receptors (TCR) with the specific antigen-bearing MHC receptors of antigen presenting cells. TCRs are surface anchored protein complexes that are comprised of two reactive antigen-recognizing chains and adjacent CD3 elements. In a TCR, the transmembrane CD3 protein complex mediates signal transduction via cytoplasmic ITAM-segments while the central antigen-recognizing chains interact with antigen presenting cells mainly with reactive loops on their tip.

Most commonly, the antigen interacting element of the TCR is dimerically built from two coupled TCRα and TCRβ chains. Each these TCRα or TCRβ chains is built from a constant segment with structural role and a variable segment that is responsible for antigen interaction. This interaction dominated by hypervariable regions situated at the loops of the respective amino acid chains that form the variable segments. Collectively, the combined TCRα and TCRβ variable segments form three antigen interacting complementarity determining regions (CDRs). If these are complimentary to the MHC/antigen complex of a presenting cell, a signal cascade is triggered that, depending on other co-stimulatory influences, can result in intracellular various processes or T-cell effector functions such as apoptosis of the antigen-presenting cell.

The wide range of T-cell specificity is achieved during their development in the thymus in which T-cells undergo different stages of maturation. In this process, they generate specific TCRs through somatic VJ or VDJ recombination of TCR alpha and beta encoding gene segments. Additionally, recombined TCR genes are further modified at their V-D-J and V-J gene junctions through exonuclease activity and addition of random non-template dependent nucleotides (N-nucleotides). The diversity creates highly variable complementarity determining regions that define the fine specificity of TCR chains.

TCRs recognize antigens in form of short peptide fragments that presented by cell surface glycoproteins referred to as major histocompatibility complexes (MHC) which are in humans encoded by human leukocyte antigen (HLA) genes and in mice by H-2 genes. In humans, two classes of MHC molecules are distinguished. MHC class I molecules are expressed on all nucleated cells and are recognized by CD8+ T-cells. MHC class II molecules that are expressed on professional antigen presenting cells, B-cells and activated T-cells and are recognized by CD₄+ T-cells. In MHC class I antigen presentation, intracellular proteins are endogenously processed in the proteasome into short peptide fragments that are typically 8-10 amino acids in length. After translocation across the endoplasmic reticulum through the transporter associated with antigen processing (TAP) and loading on MHC class I molecules, these antigens are presented for CD8+ T-cell recognition. Upon engagement of the TCRαβ dimer with the cognate antigen/MHC complex, the associated CD3 complex initiates the signaling cascade needed for T-cell activation. (Rath J. A., Arber C., Cells. 2020, 1485)

Adoptive T-cell therapy is a collective term for treatment procedures that rely on administering in vitro augmented T-cells with specificity towards a patient's tumor or virus-infected cells. There are different variants of ATT: For example, augmented T-cells can be generated by autologous harvesting and subsequent amplification/activation of T-cell fractions with adequate binding affinities. Other ATT therapies make use of T-cells that have been genetically engineered to either express optimized naturally-occurring TCRs, or, as in the case of CAR-T-cell therapy, to express artificially designed chimeric antigen-binding receptors. Naturally occurring TCRs off the advantage of recognizing endogenously processed extra- and intracellular antigens in an MHC-restricted manner on the cell surface and are therefore able to target a different array of antigens than CARs. T-cell based immunotherapy targets represent peptide epitopes derived from pathogen-associated or tumor-associated (or tumor-specific) proteins, which are presented by molecules of the major histocompatibility complex (MHC). These disease-associated antigens can be peptides derived from all protein classes, such as enzymes, receptors, transcription factors, etc. which are expressed and, as compared to unaltered cells of the same origin, usually up-regulated in cells of the respective tumor. In case the tumor is associated with a viral infection, such as with MCV or Human papilloma viruses (HPVs), immune therapy can be developed based on the virus antigens expressed by tumor cells which originate from virus infected host cells.

So far, ATT with TCR engineered T-cells has been tested in clinical trials mainly in patients with solid tumors expressing tumor associated or cancer-testis antigens. The first published clinical trial using TCR engineered T-cells targeted tumor associated antigens MART-i and gp100 in patients with metastatic melanoma and led to objective cancer regression rates of 19% or 30% respectively. However, the infused T-cells also recognized patient's melanocytes in the eye, ear and skin, thus leading to severe on-target off-tumor toxicity (Johnson et al. 2009). In another approach, sarcoma and melanoma patients were treated with TCR engineered T-cells targeting the cancer-testis antigen NY-ESO-1, which led to objective response rates of 55-80%, while no severe toxicities were observed (Rapoport et al. 2015 and Robbins et al. 2015). Finally, in another trial with TCR-engineered T-cells targeting cancer testis antigen MAGE-A3 in 5 out of 7 patients tumor regression was evident, but two patients died due to severe neurological toxicities (Morgan et al. 2013).

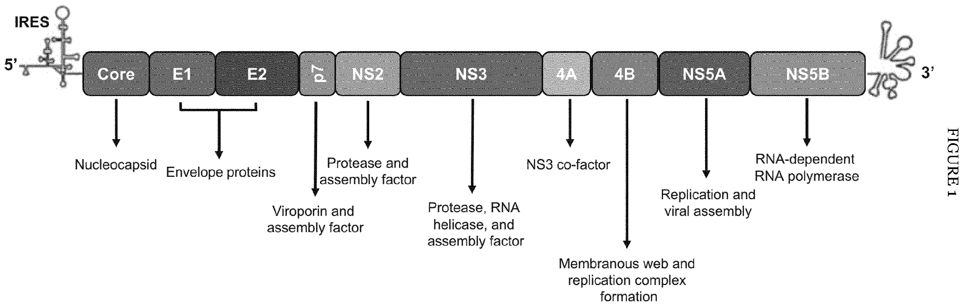

Hepatitis C Virus (HCV) is a blood-borne virus with tropism for hepatocytes and B-cells that is affecting humans and chimpanzees. It has spread rapidly since the 1930-40s mainly through contaminated blood transfusions and within high-risk groups such as health care workers, people-who-inject-drugs or men who have sex with men. (Midgard H, J Hepatol. 2016, 65(1), 33-45) HCV is a positive-sense single-stranded RNA virus belonging to the Flaviviridae family and contains a 9.6-kb genome with one single open reading frame (ORF) flanked by 5' and 3' untranslated regions (UTRs) acting as internal ribosomal entry sites (IRES) for direct translation at the host cell ribosomes. The translated HCV polyprotein is around 3000 amino acids long and is proteolytically cleaved into a range of sub-proteins described as structural (Core, E1 and E2), p7 and non-structural (NS2, NS3, NS4A, NS4B, NS5A and NS5B) (Figure 1).

Nowadays, HCV still poses a health threat. Early immune responses to HCV are difficult to analyze because of the asymptomatic nature of acute infection. Around 70% of HCV infected patients fail to clear the virus, due to early CD₄+ T-cell failure and exhaustion. Without CD₄+ T-cell help, HCV-specific CD8+ T-cell numbers rapidly decline and ultimately both subsets are deleted from peripheral blood. Remaining HCV-specific CD8+ T-cells accumulate in the liver, where chronic exposure to HCV antigen shifts them into a terminally exhausted phenotype marked by loss of effector function as well as up-regulation of inhibitory receptors like PD-1 and down-regulation of interleukin 7 receptor alpha (CD127). Approximately 20% of all chronically infected patients develop liver cirrhosis within 20-30 years, of which 1-4% develop hepatocellular carcinoma (HCC) depending on life style factors and HCV genotype. Successful immune responses on the other hand occur in 30% of the patients and are associated with sustained and vigorous HCV-specific CD4+ and CD8+ T-cell responses that are characterized by multi-specificity targeting a broad range of HCV T-cell epitopes. Accordingly, CD8+ T-cell responses that are "narrow" in terms of the targeted T-cell epitopes are associated with mutational escape and viral persistence.

In the past, the gold standard for HCV treatment has been administration of pegylated IFN-α in combination with the nucleoside inhibitor ribavirin, which leads to serious adverse effects through off-target toxicity. Nowadays, HCV infections are treated with direct-acting-antivirals (DAAs). DAAs are small molecule inhibitors specifically blocking either the NS3/4A protease, NS5B polymerase or the phosphoprotein NS5A. However, depending on the combination of DAAs used and characteristics of the treated patient group, still many (up to 15%) fail to achieve SVR through DAA treatment. This failure is associated with resistance associated substitutions (RAS) in the HCV genome that are either present at baseline in treatment-naive patients or arise through drug selective pressure. A recent study on a xenograft mouse model has demonstrated the treatment of a HCV+ engineered HCC cell line with ATT using engineered T-cells that expressed TCRs specific for the HCV epitope NS3 1406. While in this study the cells were able to delay tumor growth it is questionable, however, whether the amount of HCV antigen expressed in this genetically engineered tumor cell line reflected naturally occurring HCV antigen levels in HCV+ HCC patients. (Spear T, et al, Cancer Immunol Immunother, 2016, 65(3), 293-304).

Thus, it is an objection of the invention to provide novel adoptive cell based immune-therapies for treating Hepatitis patients. In particular, the present invention seeks to provide novel T-cell receptor molecules that are specific to HCV associated and MHC presented peptides.

### BRIEF DESCRIPTION OF THE INVENTION

Generally, and by way of brief description, the main aspects of the present invention can be described as follows:
In **a first aspect,** the invention pertains to an antigen binding protein (ABP) which, optionally, specifically binds to a Hepatitis C virus associated antigenic peptide, or a variant thereof, and wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID NOs: 3, 8, 13, 18, 23, 28, 33, 38, 43, and 48.

In **a second aspect,** the invention pertains a nucleic acid, or a nucleic acid construct (NAC), having a nucleic acid sequence encoding for an ABP of the invention.

In **a third aspect,** the invention pertains to a recombinant vector comprising the nucleic acid of the invention.

In **a fourth aspect,** the invention pertains to a recombinant host cell, comprising or expressing an ABP of the invention, or comprising a nucleic acid or vector of the invention.

In **a fifth aspect,** the invention pertains to a pharmaceutical composition comprising one or more, preferably a combination of, an ABP of the invention, or a nucleic acid or cell of the invention.

In **a sixth aspect,** the invention pertains to a compound or composition comprising any of the products of the invention, or combinations thereof, for use in medicine, in particular for use in the treatment of HCV in a subject.

In **a seventh aspect,** the invention pertains to a method of manufacturing a HCV specific antigen recognizing construct expressing cell line, comprising
- providing a suitable host cell,
- providing a genetic construct comprising a coding sequence encoding the ABP of the invention,
- introducing into said suitable host cell said genetic construct,
- expressing said genetic construct by said suitable host cell.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to an antigen binding protein (ABP) which, optionally, specifically binds to a Hepatitis C virus associated antigenic peptide, or a variant thereof, and wherein the isolated ABP comprises at least one complementarity determining region (CDR) being a CDR3 having an amino acid sequence with at least 60%, 80%, 90%, 95%, 96%, 97%, 98%, or 99% sequence identity to, or having no more than three or two, preferably no more than one amino acid substitution(s), deletion(s) or insertion(s) compared to, a sequence selected from SEQ ID NOs: 3, 8, 13, 18, 23, 28, 33, 38, 43, and 48.

In some embodiments the ABP of the invention specifically binds to a hepatitis virus antigenic peptide-HLA molecule complex, wherein the hepatitis virus antigenic peptide comprises, or alternatively consists of, a sequence derived from a Hepatitis C Virus (HCV) protein, such as a core protein or a NS3 protein. Such peptides are preferably 8 to 12, more preferably 10 +/-1 amino acids in length, and bind to an HLA class I (preferred) or class II molecule and/or induces T-cells cross-reacting with said peptide, or a pharmaceutically acceptable salt thereof, wherein said peptide is not the underlying full-length polypeptide. Preferably, the ABP of the invention when complexed with the antigenic peptide and the HLA induces / activates a T-cell immune response, optionally in the presence of one or more co-activating stimuli.

The term "antigen recognizing construct" or "antigen binding protein" are used herein interchangingly, and refer to proteins that have an ability to specifically bind other proteins, preferably immunoglobulin like proteins, such as T-cell receptors or antibodies or any known variants of this class of proteins.

The term "isolated" as used herein in the context of a polypeptide, such as an antigen recognizing construct (an example of which could be an antibody), refers to a polypeptide that is purified from proteins or polypeptides or other contaminants that would interfere with its therapeutic, diagnostic, prophylactic, research or other use. An antigen recognizing construct according to the invention may be a recombinant, synthetic or modified (non-natural) antigen binding construct. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. In this context, a "recombinant" protein/polypeptide or nucleic acid is one made using recombinant techniques. Methods and techniques for the production of recombinant nucleic acids and proteins are well known in the art.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site).); in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

In another additional or alternative embodiment were the ABP is preferably a TCR, the ABP may further comprise a CDR1 and/or a CDR2 domain sequence. Within the variable domain, CDR1 and CDR2 are found in the variable (V) region of a polypeptide chain, and CDR3 includes some of V, all of diversity (D) and joining (J) regions. CDR3 is the most variable and is the main CDR responsible for specifically and selectively recognizing an antigen. CDR1 and CDR2 sequences may be selected from a CDR sequence of a human variable chain allele.

Native alpha-beta heterodimeric TCRs have an alpha chain and a beta chain. Each chain comprises variable, joining and constant regions, and the beta chain also usually contains a short diversity region between the variable and joining regions, but this diversity region is often considered as part of the joining region. Each variable region comprises three CDRs (Complementarity Determining Regions) embedded in a framework sequence, one being the hypervariable region named CDR3. There are several types of alpha chain variable (Va) regions and several types of beta chain variable (Vβ) regions distinguished by their framework, CDR1 and CDR2 sequences, and by a partly defined CDR3 sequence. The Vα types are referred to in IMGT nomenclature by a unique TRAV number, Vβ types are referred to by a unique TRBV number. For more information on immunoglobulin antibody and TCR genes see the international ImMunoGeneTics information system^{®}, Lefranc M-P et al (Nucleic Acids Res. 2015 Jan;43(Database issue):D413-22; and http://www.imgt.org/).

Therefore, in one additional or alternative embodiment the ABP of the invention comprises CDR1, CDR2 and CDR3 sequences in a combination as provided in table 1 herein below, which display the respective variable chain allele together with the CDR3 sequence. Therefore, preferred are ABPs of the invention which comprise at least one, preferably, all three CDR sequences CDR1, CDR2 and CDR3. Preferably, an antigen recognizing construct of the invention comprises the respective CDR1 to CDR3 of one individual herein disclosed TCR variable region of the invention (see table 1 herein below and the example section).

The term "specificity" or "antigen specificity" or "specific for" a given antigen, as used herein means that the ABP can specifically bind to said antigen, preferably a HCV derived antigen, more preferably with high avidity, when said antigen is presented by HLA, preferably by HLA-A*02. For example, a TCR, as antigen recognizing construct, may be considered to have "antigenic specificity" for the HCV antigenic peptide, if T-cells expressing the TCR, or an antigen-binding variant or derivative, and contacted with a HCV derived antigenic peptide presenting HLA secrete at least about 200 pg/ml or more (e.g., 250 pg/ml or more, 300 pg/ml or more, 400 pg/ml or more, 500 pg/ml or more, 600 pg/ml or more, 700 pg/ml or more, 1000 pg ml or more, 2,000 pg/ml or more, 2,500 pg/ml or more, 5,000 pg/ml or more) of interferon γ (IFN-γ) upon co-culture with target cells pulsed with a low concentration of an antigenic peptide, such as the HCV epitopes and antigens provided herein below (e.g., about 10⁻¹¹ mol/l, 10⁻¹⁰ mol/l, 10⁻⁹ mol/l, 10⁻⁸ mol/l, 10⁻⁷ mol/l, 10⁻⁶ mol/l, 10⁻⁵ mol/l). Alternatively, or additionally, a TCR may be considered to have "antigenic specificity" for the antigenic peptide, if T-cells expressing the TCR secrete at least twice as much IFN-γ as the untransduced background level of IFN-γ upon co-culture with target cells pulsed with a low concentration of the HCV antigenic peptides. Such a "specificity" as described above can - for example - be analyzed with an ELISA.

In one alternative or additional embodiment of the invention, the antigen recognizing construct selectively binds to an antigenic peptide; preferably wherein the antigenic peptide is a protein epitope or peptide having an amino acid sequence shown in SEQ ID NO: 51 to 56, and preferably is a peptide consisting of the amino acid sequence shown in SEQ ID NO: 55 or 56, or a variant thereof, wherein the variant is an amino acid deletion, addition, insertion or substitution of not more than three, preferably two and most preferably not more than one amino acid position.

The term "selectivity" or "selective recognizing/binding" is understood to refer to the property of an ABP of the invention, such as a TCR or antibody, to selectively recognize or bind to preferably only one specific epitope and preferably shows no or substantially no cross-reactivity to another epitope. Preferably "selectivity" or "selective recognizing/binding" means that the antigen recognizing construct (e.g. a TCR) selectively recognizes or binds to preferably only one specific epitope and preferably shows no or substantially no cross-reactivity to another epitope, wherein said epitope is unique for one protein, such that the antigen recognizing construct shows no or substantially no cross-reactivity to another epitope and another protein. Surprisingly it was recognized that the preferred TCR of the invention show compared to prior art TCR a better selectivity and have a reduced off-target binding to other epitope sequences - therefore the TCR of the invention are particularly useful for treating HCV infections.

The ABP according to the invention is preferably selected from an antibody, or derivative or fragment thereof, or a T-cell receptor (TCR), or derivative or fragment thereof. A derivative or fragment of an antibody or TCR of the invention shall preferably retain the antigen binding/recognizing ability of the parent molecule, in particular its specificity and/or selectivity as explained above. Such binding functionality may be retained by the presence of a CDR3 region as defined herein. Such antigen binding specificity for a TCR is usually dependent on the HLA (MHC) presented antigenic peptide, so that the TCR forms a complex together with the peptide and the HLA molecule presenting the peptide.

In an embodiment of the invention, the inventive TCRs are able to recognize HCV antigens in a major histocompatibility complex (MHC) class I-dependent manner. "MHC class I-dependent manner," as used herein, means that the TCR elicits an immune response upon binding to HCV antigens within the context of an MHC class I molecule. The MHC class I molecule can be any MHC class I molecule known in the art, e.g., HLA-A molecules. In a preferred embodiment of the invention, the MHC class I molecule is an HLA-A*02 molecule.

The invention provides both single chain ABPs and double chain ABPs. The ABPs of the invention may be soluble or membrane bound constructs.

In an embodiment, the TCR alpha variable domain has at least one mutation relative to a TCR alpha domain shown in Table 1; and/or the TCR beta variable domain has at least one mutation relative to a TCR alpha domain shown in Table 1. In an embodiment, a TCR comprising at least one mutation in the TCR alpha variable domain and/or TCR beta variable domain has a binding affinity for, and/or a binding half-life for, an HCV peptide-HLA molecule complex, which is at least double that of a TCR comprising the unmutated TCR alpha domain and/or unmutated TCR beta variable domain.

The TCR alpha chains of the present description may further comprise a TCR alpha transmembrane domain and/or a TCR alpha intracellular domain. The TCR beta chains of the present description may further comprise a TCR beta transmembrane domain and/or a TCR beta intracellular domain.

The invention in particular provides a TCR as antigen recognizing construct, or fragment or derivative thereof. The TCR preferably is of human, which is understood as being generated from a human TCR locus and therefore comprising human TCR sequences. Furthermore, the TCR of the invention may be characterized in that it is of human origin and specifically recognizes a HCV antigen of the invention.

Another embodiment of the invention additionally or alternatively provides the antigen recognizing construct described above, which induces an immune response, preferably wherein the immune response is characterized by an increase in interferon (IFN) γ levels.

TCRs of the invention may be provided as single chain α or β, or γ and δ, molecules, or alternatively as double chain constructs composed of both the α and β chain, or γ and δ chain.

The antigen recognizing construct of the invention may comprise a TCR α or γ chain; and/or a TCR β or δ chain; wherein the TCR α or γ chain comprises a CDR3 having at least 50%, 60%, 70%, 80%, 90%, 95%, 98%, 99%, or 100% sequence identity to an amino acid sequence selected from SEQ ID NOs. 3, 13, 23, 33, or 43, and/or wherein the TCR β or δ chain comprises a CDR3 having at least 50%, 60%, 70%, 80%, 90%, 95%, 98%, 99%, or 100% sequence identity to an amino acid sequence selected from SEQ ID NOs. 8, 18, 28, 38, or 48. Also preferred is a ABP comprising a TCR α or γ chain; and/or a TCR β or δ chain; wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID NOs: 3, 13, 23, 33, and 43; and/or wherein the TCR β or δ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID NOs: 8, 18, 28, 38, and 48.

Most preferably, in some additional embodiments, wherein the disclosure refers to antigen recognizing constructs comprising any one, two or all of the CDR1 to CDR3 regions of the herein disclosed TCR chains (see table 1), such antigen recognizing constructs may be preferred, which comprise the respective CDR sequence of the invention with not more than three, two, and preferably only one, modified amino acid residues. A modified amino acid residue may be selected from an amino acid insertion, deletion or substitution. Most preferred is that the three, two, preferably only one modified amino acid residue is the first or last amino acid residue of the respective CDR sequence. If the modification is a substitution then it is preferable in some embodiments that the substitution is a conservative amino acid substitution.

If the antigen recognizing construct of the invention is composed of at least two amino acid chains, such as a double chain TCR, or antigen binding fragment thereof, the antigen recognizing construct may comprises in a first polypeptide chain the amino acid sequence according to SEQ ID NO: 3, and in a second polypeptide chain the amino acid sequence according to SEQ ID NO: 8; or in a first polypeptide chain the amino acid sequence according to SEQ ID NO: 13, and in a second polypeptide chain the amino acid sequence according to SEQ ID NO: 18; or in a first polypeptide chain the amino acid sequence according to SEQ ID NO: 23, and in a second polypeptide chain the amino acid sequence according to SEQ ID NO: 28 or in a first polypeptide chain the amino acid sequence according to SEQ ID NO: 33, and in a second polypeptide chain the amino acid sequence according to SEQ ID NO: 38 or in a first polypeptide chain the amino acid sequence according to SEQ ID NO: 43, and in a second polypeptide chain the amino acid sequence according to SEQ ID NO: 48.

Any one of the aforementioned double chain TCR, or antigen binding fragments thereof, are preferred TCR of the present invention. In some embodiments, the CDR3 of the double chain TCR of the invention may be mutated. Mutations of the CDR3 sequences as shown in table 1 as provided above preferably include a substitution, deletion, addition, or insertion of not more than three, preferably two, and most preferably not more than one amino acid residue. In some embodiments, the first polypeptide chain may be a TCR α or γ chain, and the second polypeptide chain may be a TCR β or δ chain. Preferred is the combination of an αβ or γδ TCR.

The TCR, or the antigen binding fragment thereof, is in some embodiments composed of a TCR α and a TCR β chain, or γ and δ chain. Such a double chain TCR comprises within each chain variable regions, and the variable regions each comprise one CDR1, one CDR2 and one CDR3 sequence. The TCRs comprises the CDR1 to CDR3 sequences as comprised in the variable chain amino acid sequence of SEQ ID NO: 5 and SEQ ID NO: 10, or SEQ ID NO: 15 and SEQ ID NO: 20; or SEQ ID NO: 25 and SEQ ID NO: 30, or SEQ ID NO: 35 and SEQ ID NO: 40, or SEQ ID NO: 45 and SEQ ID NO: 50.

Some embodiments of the invention pertain to a TCR, or a fragment thereof, composed of a TCR α and a TCR β chain, wherein said TCR comprises the variable region sequences having at least 50%, 60%, 70%, 80%, 90%, 95%, 98%, 99%, or preferably 100% sequence identity to the amino acid sequence selected from the α and β chain according to SEQ ID NO: 5 and 10 respectively, or 15 and 20 respectively; or 25 and 30 respectively; or 35 and 40 respectively; or 45 and 50 respectively.

The inventive TCRs may further comprise a constant region derived from any suitable species, such as any mammal, e.g., human, rat, monkey, rabbit, donkey, or mouse. In an embodiment of the invention, the inventive TCRs further comprise a human constant region. In some preferred embodiments, the constant region of the TCR of the invention may be slightly modified, for example, by the introduction of heterologous sequences, preferably mouse sequences, which may increase TCR expression and stability.

The TCR α or γ chain of the invention may further comprise a CDR1 having at least 50%, 60%, 70%, 80%, 90%, 95%, 98%, 99%, or 100% sequence identity to, or having no more than three or two, preferably no more than one amino acid substitution(s), deletion(s) or insertion(s) compared to, a sequence selected from SEQ ID NOs: 1, 11, 21, 31, and 41; and/or a CDR2 having an amino acid sequence with at least 80% sequence identity to, or having no more than three or two, preferably no more than one amino acid substitution(s), deletion(s) or insertion(s) compared to, a sequence selected from SEQ ID NOs: 2, 12, 22, 32, and 42.

According to the invention the TCR β or δ chain may further comprise a CDR1 having at least 50%, 60%, 70%, 80%, 90%, 95%, 98%, 99%, or 100% sequence identity to, or having no more than three or two, preferably no more than one amino acid substitution(s), deletion(s) or insertion(s) compared to, a sequence selected from SEQ ID NOs: 6, 16, 26, 36, and 46; and/or a CDR2 having an amino acid sequence with at least 80% sequence identity to, or having no more than three or two, preferably no more than one amino acid substitution(s), deletion(s) or insertion(s) compared to, a sequence selected from SEQ ID NOs: 7, 17, 27, 37, and 47.

The antigen recognizing construct may in a further embodiment comprise a binding fragment of a TCR, and wherein said binding fragment comprises CDR1 to CDR3, optionally selected from the CDR1 to CDR3 sequences having the amino acid sequences of SEQ ID NOs. 1, 2, 3, or 6, 7, 8 or 11, 12, 13, or 16, 17, 18 or 21, 22, 23, or 26, 27, 28 or 31, 32, 33, or 36, 37, 38, or 41, 42, 43, or 46, 47, 48, each CDR independently having no more than three or two, preferably no more than one amino acid substitution(s), deletion(s) or insertion(s) compared to these sequences.

In further embodiments of the invention the antigen recognizing construct as described herein elsewhere is a TCR, or a fragment thereof, composed of at least one TCR α and one TCR β chain sequence, wherein said TCR α chain sequence comprises the CDR1 to CDR3 sequences having the amino acid sequences of SEQ ID NO: 1 to 3, and said TCR β chain sequence comprises the CDR1 to CDR3 sequences having the amino acid sequences of SEQ ID NO: 6 to 8; or wherein said TCR α chain sequence comprises the CDR1 to CDR3 sequences having the amino acid sequences of SEQ ID NO: 11 to 13, and said TCR β chain sequence comprises the CDR1 to CDR3 sequences having the amino acid sequences of SEQ ID NO: 16 to 18; or wherein said TCR α chain sequence comprises the CDR1 to CDR3 sequences having the amino acid sequences of SEQ ID NO: 21 to 23, and said TCR β chain sequence comprises the CDR1 to CDR3 sequences having the amino acid sequences of SEQ ID NO: 26 to 28; or wherein said TCR α chain sequence comprises the CDR1 to CDR3 sequences having the amino acid sequences of SEQ ID NO: 31 to 33, and said TCR β chain sequence comprises the CDR1 to CDR3 sequences having the amino acid sequences of SEQ ID NO: 36 to 38; or wherein said TCR α chain sequence comprises the CDR1 to CDR3 sequences having the amino acid sequences of SEQ ID NO: 41 to 43, and said TCR β chain sequence comprises the CDR1 to CDR3 sequences having the amino acid sequences of SEQ ID NO: 46 to 48; each CDR independently having no more than three or two, preferably no more than one amino acid substitution(s), deletion(s) or insertion(s) compared to these sequences.

In further embodiments of the invention the antigen recognizing construct as described herein before is a TCR, or a fragment thereof, comprising at least one TCR α and one TCR β chain sequence, wherein said TCR α chain sequence comprises a variable region sequence having the amino acid sequence of SEQ ID NO: 4, and wherein said TCR β chain sequence comprises a variable region sequence having the amino acid sequence of SEQ ID NO: 9; or wherein said TCR α chain sequence comprises a variable region sequence having the amino acid sequence of SEQ ID NO: 14, and wherein said TCR β chain sequence comprises a variable region sequence having the amino acid sequence of SEQ ID NO: 19; or wherein said TCR α chain sequence comprises a variable region sequence having the amino acid sequence of SEQ ID NO: 24, and wherein said TCR β chain sequence comprises a variable region sequence having the amino acid sequence of SEQ ID NO: 29; or wherein said TCR α chain sequence comprises a variable region sequence having the amino acid sequence of SEQ ID NO: 34, and wherein said TCR β chain sequence comprises a variable region sequence having the amino acid sequence of SEQ ID NO: 39; or wherein said TCR α chain sequence comprises a variable region sequence having the amino acid sequence of SEQ ID NO: 44, and wherein said TCR β chain sequence comprises a variable region sequence having the amino acid sequence of SEQ ID NO: 49; each variable domain sequence having no more than 20, preferably no more than 15, more preferably no more than 10, 5, three or two, preferably no more than one amino acid substitution(s), deletion(s) or insertion(s) compared to these sequences.

In further embodiments of the invention the antigen recognizing construct as described herein before is a TCR, or a fragment thereof, further comprising a TCR constant region having at least 50%, 60%, 70%, 80%, 90%, 95%, 98%, 99%, or 100% sequence identity to an amino acid sequence selected from a human TCR constant domain.

As used herein, the term "murine" or "human," when referring to an antigen recognizing construct, or a TCR, or any component of a TCR described herein (e.g., complementarity determining region (CDR), variable region, constant region, α chain, and/or β chain), means a TCR (or component thereof), which is derived from a mouse or a human unrearranged TCR locus, respectively.

In an embodiment of the invention, chimeric TCR are provided, wherein the TCR chains comprise sequences from multiple species. Preferably, a TCR of the invention may comprise an α chain comprising a human variable region of an α chain and, for example, a murine constant region of a murine TCR α chain.

In one embodiment, the TCR of the invention is a human TCR comprising human variable regions according to the above embodiments and human constant regions.

In some embodiments the antigen recognizing construct is murinized or humanized. These terms are used when amino acid sequences from a foreign species are introduced into a construct of the invention.

The TCR of the invention may be provided as a single chain TCR (scTCR). A scTCR can comprise a polypeptide of a variable region of a first TCR chain (e.g., an alpha chain) and a polypeptide of an entire (full-length) second TCR chain (e.g., a beta chain), or vice ver-sa. Furthermore, the scTCR can optionally comprise one or more linkers which join the two or more polypeptides together. The linker can be, for instance, a peptide, which joins together two single chains, as described herein. Also provided is such a scTCR of the invention, which is fused to a human cytokine, such as IL-2, IL-7 or IL-15.

The ABP according to the invention can also be provided in the form of a multimeric complex, comprising at least two TCR or scTCR molecules, wherein said TCR or scTCR molecules are each fused to at least one biotin moiety, or other interconnecting molecule/linker, and wherein said TCR or scTCRs are interconnected by biotin-streptavidin interaction to allow the formation of said multimeric complex. Similar approaches known in the art for the generation of multimeric TCR are also possible and included in this disclosure. Also provided are multimeric complexes of a higher order, comprising more than two scTCR of the invention.

For the purposes of the present invention, a TCR is a moiety having at least one TCR alpha or gamma and/or TCR beta or delta variable domain. Generally, they comprise both a TCR alpha variable domain and a TCR beta variable domain, alternatively both a TCR gamma variable domain and a TCR delta variable domain. They may be αβ/γδ hetero-dimers or may be in single chain format. For use in adoptive therapy, an αβ or γδ heterodimeric TCR may, for example, be transfected as full-length chains having both cytoplasmic and transmembrane domains. If desired, an introduced disulfide bond between residues of the respective constant domains may be present.

In a preferred embodiment, the ABP of the invention is a human TCR, or fragment or derivative thereof. A human TCR or fragment or derivative thereof is a TCR, which comprises over 50% of the corresponding human TCR sequence. Preferably, only a small part of the TCR sequence is of artificial origin or derived from other species. It is known, however, that chimeric TCRs, e.g. derived from human origin with murine sequences in the constant domains, are advantageous. Particularly preferred are, therefore, TCRs in accordance with the present invention, which contains murine sequences in the extracellular part of their constant domains.

Thus, it is also preferred that the inventive ABP is able to recognize its respective antigen in a human leucocyte antigen (HLA) dependent manner, preferably in a HLA-A02 dependent manner. The term "HLA dependent manner" in the context of the present invention means that the antigen recognizing construct binds to the antigen only in the event that the antigenic peptide is presented by said HLA.

The antigen recognizing construct in accordance with the invention in one embodiment preferably induces an immune response, preferably wherein the immune response is characterized by the increase in interferon (IFN) γ levels.

Also provided by the invention is a polypeptide comprising a functional portion of any of the TCRs (or functional variants thereof) described herein, for examples, of any one of the TCRs selected from the TCR as provided in the example section and table 1. The term "polypeptide" as used herein includes oligopeptides and refers to a single chain of amino acids connected by one or more peptide bonds. With respect to the inventive polypeptides, the functional portion can be any portion comprising contiguous amino acids of the TCR (or functional variant thereof), of which it is a part, provided that the functional portion specifically binds to the HCV antigen, preferably as disclosed herein in table 2 (SEQ ID NOs: 51 to 56). The term "functional portion" when used in reference to a TCR (or functional variant thereof) refers to any part or fragment of the TCR (or functional variant thereof) of the invention, which part or fragment retains the biological activity of the TCR (or functional variant thereof), of which it is a part (the parent TCR or parent functional variant thereof). Functional portions encompass, for example, those parts of a TCR (or functional variant thereof) that retain the ability to specifically bind to the HCV antigen (in an HLA dependent manner), or detect, treat, or prevent HCV infections, to a similar extent, the same extent, or to a higher extent, as the parent TCR (or functional variant thereof). In reference to the parent TCR (or functional variant thereof), the functional portion can comprise, for instance, about 10%, 25%, 30%, 50%, 68%, 80%, 90%, 95%, or more, of the parent TCR variable sequences (or functional variant thereof).

The functional portion can comprise additional amino acids at the amino or carboxy terminus of the portion, or at both termini, in which additional amino acids are not found in the amino acid sequence of the parent TCR or functional variant thereof. Desirably, the additional amino acids do not interfere with the biological function of the functional portion, e.g., specifically binding to the HCV antigens; and/or having the ability to detect cancer, treat or prevent cancer, etc. More desirably, the additional amino acids enhance the biological activity, as compared to the biological activity of the parent TCR or functional variant thereof.

The polypeptide can comprise a functional portion of either or both of the α and β chains of the TCRs or functional variant thereof of the invention, such as a functional portion comprising one of more of CDR1, CDR2, and (preferably) CDR3 of the variable region(s) of the α chain and/or β chain of a TCR or functional variant thereof of the invention. In an embodiment of the invention, the polypeptide can comprise a functional portion comprising the amino acid sequence of SEQ ID NO: 3, 8, 13, 18, 23, 28, 33, 38, 43, or 48 (CDR3 of the variable regions of the TCR of the invention), or a combination thereof. In an embodiment of the invention, the inventive polypeptide can comprise, for instance, the variable region of the inventive TCR or functional variant thereof comprising a combination of the CDR regions set forth above. In this regard, the polypeptide can comprise the amino acid sequence of any of SEQ ID NO: 5, 10, 15, 20, 25, 30, 35, 40, 45, and 50 (the variable regions of an α or β chain of the TCR of the invention).

In some instances, the construct of the invention may comprise one or two polypeptide chains comprising a sequence, or a sequence encoded by a nucleic acid sequence, according to any one of the SEQ ID NO: 1 to 50 (CDR sequences, constant and variable regions and nucleic acid sequences), or functional fragments thereof, and further comprise(s) other amino acid sequences, e.g., an amino acid sequence encoding an immunoglobulin or a portion thereof, then the inventive protein can be a fusion protein. In this regard, the invention also provides a fusion protein comprising at least one of the inventive polypeptides described herein along with at least one other polypeptide. The other polypeptide can exist as a separate polypeptide of the fusion protein, or can exist as a polypeptide, which is expressed in frame (in tandem) with one of the inventive polypeptides described herein. The other polypeptide may include any peptidic or proteinaceous molecule, or a portion thereof, including, but not limited to an immunoglobulin, CD3, CD4, CD8, an MHC molecule, a CD1 molecule, e.g., CD1a, CD1b, CD1c, CD1d, etc.

The fusion protein can comprise one or more copies of the inventive polypeptide and/or one or more copies of the other polypeptide. For instance, the fusion protein can comprise 1, 2, 3, 4, 5, or more, copies of the inventive polypeptide and/or of the other polypeptide. Suitable methods of making fusion proteins are known in the art, and include, for example, recombinant methods. In some embodiments of the invention, the TCRs (and functional portions and functional variants thereof), polypeptides, and proteins of the invention may be expressed as a single protein comprising a linker peptide linking the α chain and the β chain, and linking the γ chain and the δ chain. In this regard, the TCRs (and functional variants and functional portions thereof), polypeptides, and proteins of the invention comprising the amino acid sequences of the variable regions of the TCR of the invention and may further comprise a linker peptide. The linker peptide may advantageously facilitate the expression of a recombinant TCR (including functional portions and functional variants thereof), polypeptide, and/or protein in a host cell. The linker peptide may comprise any suitable amino acid sequence. Linker sequences for single chain TCR constructs are well known in the art. Such a single chain construct may further comprise one, or two, constant domain sequences. Upon expression of the construct including the linker peptide by a host cell, the linker peptide may also be cleaved, resulting in separated α and β chains, and separated γ and δ chain.

As already mentioned above, the binding functionality of the TCR of the invention may be provided in the framework of an antibody. For example, CDR sequences of the TCR of the invention, possibly including additional 3, 2 or 1 N and/or c terminal framework residues, may be directly grafted into an antibody variable heavy/light chain sequence. The term "antibody" in its various grammatical forms is used herein to refer to immunoglobulin molecules and immunologically active portions of immunoglobulin molecules, i.e., molecules that contain an antigen-binding site or a paratope. Such molecules are also referred to as "antigen binding fragments" of immunoglobulin molecules. The invention further provides an antibody, or antigen binding portion thereof, which specifically binds to the antigens described herein. The antibody can be any type of immunoglobulin that is known in the art. For instance, the antibody can be of any isotype, e.g., IgA, IgD, IgE, IgG, IgM, etc. The antibody can be monoclonal or polyclonal. The antibody can be a naturally-occurring antibody, e.g., an antibody isolated and/or purified from a mammal, e.g., mouse, rabbit, goat, horse, chicken, hamster, human, etc. Alternatively, the antibody can be a genetically-engineered antibody, e.g., a humanized antibody or a chimeric antibody. The antibody can be in monomeric or polymeric form.

The term "antibody" includes, but is not limited to, genetically engineered or otherwise modified forms of immunoglobulins, such as intrabodies, chimeric antibodies, fully human antibodies, humanized antibodies (e.g. generated by "CDR-grafting"), antibody fragments, and heteroconjugate antibodies (e.g., bispecific antibodies, diabodies, triabodies, tetra-bodies, etc.). The term "antibody" includes cys-diabodies and minibodies. Thus, each and every embodiment provided herein in regard to "antibodies", or "antibody like constructs" is also envisioned as, bispecific antibodies, diabodies, scFv fragments, chimeric antibody receptor (CAR) constructs, diabody and/or minibody embodiments, unless explicitly denoted otherwise. The term "antibody" includes a polypeptide of the immunoglobulin family or a polypeptide comprising fragments of an immunoglobulin that is capable of non-covalently, reversibly, and in a specific manner binding a corresponding antigen, preferably a HCV antigenic peptide of the invention, as disclosed herein. An exemplary antibody structural unit comprises a tetramer. In some embodiments, a full-length antibody can be composed of two identical pairs of polypeptide chains, each pair having one "light" and one "heavy" chain (connected through a disulfide bond). Antibody structure and isotypes are well known to the skilled artisan (for example from Janeway's Immuno-biology, 9th edition, 2016).

The recognized immunoglobulin genes of mammals include the kappa, lambda, alpha, gamma, delta, epsilon, and mu constant region genes, as well as the myriad immuno-globulin variable region genes (for more information on immunoglobulin genes see the international ImMunoGeneTics information system^{®}, Lefranc M-P et al, Nucleic Acids Res. 2015 Jan;43(Database issue):D413-22; and http://www.imgt.org/). For full-length chains, the light chains are classified as either kappa or lambda. For full-length chains, the heavy chains are classified as gamma, mu, alpha, delta, or epsilon, which in turn de-fine the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (VL) and variable heavy chain (VH) refer to these regions of light and heavy chains respectively. As used in this invention, an "antibody" encompasses all variations of antibody and fragments thereof. Thus, within the scope of this concept are full length antibodies, chimeric antibodies, humanized antibodies, single chain antibodies (scFv), Fab, Fab', and multimeric versions of these fragments (e.g., F(ab')2) with the same, essentially the same or similar binding specificity. In some embodiments, the antibody binds specifically to a HCV peptide of the invention. Preferred antigen recognizing constructs according to the invention include an antibody heavy chain, preferably the variable domain thereof, or an antigen binding fragment thereof, and/or an antibody light chain, preferably the variable domain thereof, or an antigen binding fragment thereof. Similarly, disulfide-stabilized variable region fragments (dsFv) can be prepared by recombinant DNA technology, antibody fragments of the invention, however, are not limited to these exemplary types of antibody fragments. Also, the antibody, or antigen binding portion thereof, can be modified to comprise a detectable label, such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles). In some instances, the TCR CDR3 sequence may be slightly modified, but preferably by not more than 3 amino acid residues, preferably only two and most preferably only one amino acid position, as compared to the CDR3 sequences provided in SEQ ID NOs: 3, 8, 13, 18, 23, 28, 33, 38, 43, and 48. Preferably, the antibodies comprise the CDR3, preferably all of CDR1 to CDR3 regions in the combination, as indicated for the TCR of the invention in table 1, in each case independently, optionally with not more than three or two, preferably one, amino acid substitution(s), insertion(s) and/or deletion(s) compared to these sequences.

Suitable methods of making antibodies are known in the art. For instance, standard hybridoma methods are described in, e.g., Kohler and Milstein, Eur. J. Immunol, 5, 51 1-519 (1976), Harlow and Lane (eds.), Antibodies: A Laboratory Manual, CSH Press (1988), and C.A. Janeway et al. (eds.), Immunobiology, 8 Ed., Garland Publishing, New York, NY (201 1)). Alternatively, other methods, such as EBV-hybridoma methods (Haskard and Archer, J. Immunol. Methods, 74(2), 361-67 (1984), and Roder et al, Methods Enzymol, 121, 140-67 (1986)), and bacteriophage vector expression systems (see, e.g., Huse et al., Science, 246,1275-81 (1989)) are known in the art. Further, methods of producing anti-bodies in non-human animals are described in, e.g., U.S. Patents 5,545,806, 5,569,825, and 5,714,352, and U.S. Patent Application Publication No. 2002/0197266.

Some embodiments of the invention also pertain to TCRs, or functional fragments and polypeptides thereof, which are soluble TCRs. As used herein, the term "soluble T-cell receptor" refers to heterodimeric truncated variants of native TCRs, which comprise extracellular portions of the TCR α-chain and β-chain, for example linked by a disulfide bond, but which lack the transmembrane and cytosolic domains of the native protein. The terms "soluble T-cell receptor α-chain sequence and soluble T-cell receptor β-chain sequence" refer to TCR α-chain and β-chain sequences that lack the transmembrane and cytosolic domains. The sequence (amino acid or nucleic acid) of the soluble TCR α -chain and β-chains may be identical to the corresponding sequences in a native TCR or may comprise variant soluble TCR α-chain and β-chain sequences, as compared to the corresponding native TCR sequences. The term "soluble T-cell receptor" as used herein encompasses soluble TCRs with variant or non-variant soluble TCR α-chain and β-chain sequences. The variations may be in the variable or constant regions of the soluble TCR α-chain and β-chain sequences and can include, but are not limited to, amino acid deletion, insertion, substitution mutations as well as changes to the nucleic acid sequence, which do not alter the amino acid sequence. Soluble TCR of the invention in any case retain the binding functionality of their parent molecules.

In **a second aspect,** the invention pertains a nucleic acid, or a nucleic acid construct (NAC), having a nucleic acid sequence encoding for an ABP of the invention.

The above problem is further solved by a nucleic acid encoding for an antigen recognizing construct or ABP of the invention, or any of the aforementioned protein or polypeptide constructs. The nucleic acid preferably (a) has a strand encoding for an antigen recognizing construct according to the invention; (b) has a strand complementary to the strand in (a); or (c) has a strand that hybridizes under stringent conditions with a molecule as described in (a) or (b). Stringent conditions are known to the person of skill in the art, specifically from Sambrook et al, "Molecular Cloning". In addition to that, the nucleic acid optionally has further sequences, which are necessary for expressing the nucleic acid sequence corresponding to the protein, specifically for expression in a mammalian/human cell. The nucleic acid used can be contained in a vector suitable for allowing expression of the nucleic acid sequence corresponding to the peptide in a cell. However, the nucleic acids can also be used to transform an antigen-presenting cell, which may not be restricted to classical antigen-presenting cells, such as dendritic cells, in such a way that they themselves produce the corresponding proteins on their cellular surface.

In some embodiments, the polypeptides of the antigen recognizing constructs or ABP can be encoded by nucleic acids and expressed in vivo or in vitro. Thus, in some embodiments, a nucleic acid encoding an antigen recognizing construct or ABP is provided. In some embodiments, the nucleic acid encodes one part or monomer of an antigen recognizing construct or ABP of the invention (for example one of two chains of a TCR of the invention), and/or another nucleic acid encodes another part or monomer of an antigen recognizing con-struct or ABP of the invention (for example the other of two chains of the TCR). In some embodiments, the nucleic acid encodes two or more antigen recognizing construct or ABP polypeptide chains, for example, at least 2 TCR chains. Nucleic acids encoding multiple antigen recognizing construct or ABP chains can include nucleic acid cleavage sites between at least two chain sequences, can encode transcription or translation start site between two or more chains sequences, and/or can encode proteolytic target sites between two or more antigen recognizing construct or ABP chains.

By "nucleic acid" as used herein includes "polynucleotide," "oligonucleotide," and "nucleic acid molecule," and generally means a polymer of DNA or RNA, which can be single-stranded or double-stranded, synthesized or obtained (e.g., isolated and/or purified) from natural sources, which can contain natural, non-natural or altered nucleotides, and can contain a natural, non-natural or altered internucleotide linkage, such as a phosphoroamidate linkage or a phosphorothioate linkage, instead of the phosphodiester found between the nucleotides of an unmodified oligonucleotide.

Preferably, the nucleic acids of the invention are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be in vitro replication or in vivo replication. The nucleic acid can comprise any nucleotide sequence, which encodes any of the TCRs, polypeptides, or proteins, or functional portions or functional variants thereof described herein.

In a **third aspect,** the invention pertains to a recombinant vector comprising the nucleic acid of the invention.

Furthermore, the invention provides a vector comprising a nucleic acid in accordance to the invention as described above. Desirably, the vector is an expression vector or a recombinant expression vector. The term "recombinant expression vector" refers in context of the present invention to a nucleic acid construct that allows for the expression of an mRNA, protein or polypeptide in a suitable host cell. The recombinant expression vector of the invention can be any suitable recombinant expression vector, and can be used to transform or transfect any suitable host. Suitable vectors include those designed for propagation and expansion or for expression or both, such as plasmids and viruses. Examples of animal expression vectors include pEUK-Cl, pMAM, and pMAMneo. Preferably, the recombinant expression vector is a viral vector, e.g., a retroviral vector. The recombinant expression vector comprises regulatory sequences, such as transcription and translation initiation and termination codons, which are specific to the type of host cell (e.g., bacterium, fungus, plant, or animal), into which the vector is to be introduced and in which the expression of the nucleic acid of the invention may be performed. Furthermore, the vector of the invention may include one or more marker genes, which allow for selection of transformed or transfected hosts. The recombinant expression vector can comprise a native or normative promoter operably linked to the nucleotide sequence encoding the constructs of the invention, or to the nucleotide sequence, which is complementary to or which hybridizes to the nucleotide sequence encoding the constructs of the invention. The selections of promoters include, e.g., strong, weak, inducible, tissue-specific and developmental-specific promoters. The promoter can be a non-viral promoter or a viral promoter. The inventive recombinant expression vectors can be designed for either transient expression, for stable expression, or for both. Also, the recombinant expression vectors can be made for constitutive expression or for inducible expression.

Such vectors can either contain nucleic acids encoding one or both TCR chains, therefore may comprise a sequence encoding for the alpha and/or beta chain of the ABP of the invention.

In **a fourth aspect,** the invention pertains to a recombinant host cell, comprising or expressing an ABP of the invention, or comprising a nucleic acid or vector of the invention.

The invention also pertains to a host cell comprising an antigen recognizing construct or ABP in accordance with the invention. Specifically, the host cell of the invention comprises a nucleic acid, or a vector as described herein above. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing a recombinant TCR, polypeptide, or protein, the host cell is preferably a mammalian cell. Most preferably, the host cell is a human cell. While the host cell can be of any cell type, can originate from any type of tissue, and can be of any developmental stage, the host cell preferably is a peripheral blood leukocyte (PBL) or a peripheral blood mononuclear cell (PBMC). More preferably, the host cell is a T-cell. The T-cell can be any T-cell, such as a cultured T-cell, e.g., a primary T-cell, or a T-cell from a cultured T-cell line, e.g., Jurkat, SupTi, etc., or a T-cell obtained from a mammal, preferably a T-cell or T-cell precursor from a human patient. If obtained from a mammal, the T-cell can be obtained from numerous sources, including but not limited to blood, bone marrow, lymph node, the thymus, or other tissues or fluids. T-cells can also be enriched for or purified. Preferably, the T-cell is a human T-cell. More preferably, the T-cell is a T-cell isolated from a human. The T-cell can be any type of T-cell and can be of any developmental stage, including but not limited to, CD4-positive and/or CD8-positive, CD4-positive helper T-cells, e.g., Th1 and Th2 cells, CD8-positive T-cells (e.g., cytotoxic T-cells), tumor infiltrating cells (TILs), memory T-cells, naive T-cells, and the like. Preferably, the T-cell is a CD8-positive T-cell or a CD4-positive T-cell.

Preferably, the host cell of the invention is a lymphocyte, preferably, a T lymphocyte, such as a CD4-positive or CD8-positive T-cell. The host cell furthermore preferably is a HCV antigen reactive T-cell specific for HCV antigen presenting cells.

In **a fifth aspect,** the invention pertains to a pharmaceutical composition comprising one or more, preferably a combination of, an ABP of the invention, or a nucleic acid or cell of the invention.

Thus also provided is a pharmaceutical composition, comprising any of the herein described products of the invention and TCR materials of the invention, specifically any proteins, nucleic acids or host cells. In a preferred embodiment the pharmaceutical composition is for immune therapy, preferably adoptive cell therapy.

Preferably, the inventive TCR material is administered by injection, e.g., intravenously. When the inventive TCR material is a host cell expressing the inventive TCR (or functional variant thereof), the pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment, the pharmaceutically acceptable carrier is supplemented with human serum albumen.

For purposes of the invention, the amount or dose (e.g., numbers of cells when the inventive TCR material is one or more cells) of the inventive TCR material administered may be sufficient to affect, e.g., a therapeutic or prophylactic response, in the subject or animal over a reasonable time frame. For example, the dose of the inventive TCR material should be sufficient to bind to a cancer antigen, or detect, treat or prevent cancer in a period of from about 2 hours or longer, e.g., 12 to 24 or more hours, from the time of administration. In certain embodiments, the time period could be even longer. The dose will be determined by the efficacy of the particular inventive TCR material and the condition of the animal (e.g., human), as well as the body weight of the animal (e.g., human) to be treated.

It is contemplated that the inventive pharmaceutical compositions, antigen recognizing constructs, TCRs (including functional variants thereof), polypeptides, proteins, nucleic acids, recombinant expression vectors, host cells, or populations of cells can be used in methods of treating or preventing a HCV infection, or condition associated with a HCV-positive cell. The inventive TCRs (and functional variants thereof) are believed to bind specifically to the HCV antigens of the invention, such that the TCR (or related inventive polypeptide or protein and functional variants thereof), when expressed by or on a cell, such as a T-cell, is able to mediate an immune response against a target cell expressing the HCV antigen of the invention, preferably presenting HCV antigenic peptides via MHC I on the surface of said target cell. In this regard, the invention provides a method of treating or preventing a condition, in particular an acute or chronic HCV infection, in a mammalian subject, comprising administering to the mammal any of the pharmaceutical compositions, isolated ABPs, or combinations of ABPs of the invention, in particular TCRs (and functional variants thereof), polypeptides, or proteins described herein, any nucleic acid or recombinant expression vector comprising a nucleotide sequence encoding any of the TCRs (and functional variants thereof), polypeptides, proteins described herein, or any host cell or population of cells comprising a nucleic acid or recombinant vector, which encodes any of the constructs of the invention (and functional variants thereof), polypeptides, or proteins described herein, in an amount effective to treat or prevent the condition in the mammal, wherein the condition is preferably a HCV infection or any disease or condition associated with such infection.

Examples of pharmaceutically acceptable carriers or diluents useful in the present invention include stabilizers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

In **a sixth aspect,** the invention pertains to a compound or composition comprising any of the products of the invention, or combinations thereof, for use in medicine, in particular for use in the treatment of HCV in a subject.

The constructs, proteins, TCRs antibodies, polypeptides and nucleic acids of the invention are in particular for use in immune therapy, preferably, in adoptive T-cell therapy. The administration of the compounds of the invention can, for example, involve the infusion of T-cells of the invention into said patient. Preferably, such T-cells are autologous T-cells of the patient and in vitro transduced with a nucleic acid or antigen recognizing construct of the present invention.

A HCV infection associated disease treatable in context of the present invention is also a cancerous disease associated with a, for example chronic, HCV infection. Hence, the products of the invention may also be used for the treatment or prevention of a tumor disease in a subject, preferably wherein the tumor is a liver tumor such as HCC.

In particular, preferred is a treatment of a patient that received or receives treatment with another HCV therapeutic, for example wherein the other HCV therapeutic is selected from a protease inhibitor such as one described herein in Table 3. It was surprisingly found that the TCR of the invention, and in particular HCV-T001 (as well as any variant thereof comprising the CDRs of the TCR of the invention), recognizes HCV epitopes that are not affected by typical protease inhibitor therapy induced mutations/resistance. Therefore, in a preferred embodiment, the disease is a protease inhibitor resistant HCV infection, preferably wherein the resistance is a resistance induced by treatment with Asunaprevir, Grazoprevir, Simeprevir, and/or Vaniprevir

The inventive antigen recognizing constructs, ABPs, such as TCRs, polypeptides, proteins (including functional variants thereof), nucleic acids, recombinant expression vectors, host cells (including populations thereof), and antibodies (including antigen binding portions thereof), all of which are collectively referred to as "inventive TCR materials" hereinafter, can be formulated into a composition, such as a pharmaceutical composition. In this regard, the invention provides a pharmaceutical composition comprising any of the antigen recognizing constructs, TCRs, polypeptides, proteins, functional portions, functional variants, nucleic acids, expression vectors, host cells (including populations thereof), and antibodies (including antigen binding portions thereof) described herein, and a pharmaceutically acceptable carrier, excipient and/or stabilizer. The inventive pharmaceutical compositions containing any of the inventive TCR materials can comprise more than one inventive TCR material, e.g., a polypeptide and a nucleic acid, or two or more different TCRs (including functional portions and functional variants thereof). Alternatively, the pharmaceutical composition can comprise an inventive TCR material in combination with another pharmaceutically active agent(s) or drug(s), such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. Preferably, the carrier is a pharmaceutically acceptable carrier. With respect to pharmaceutical compositions, the carrier can be any of those conventionally used for the particular inventive TCR material under consideration. Such pharmaceutically acceptable carriers are well-known to those skilled in the art and are readily available to the public. It is preferred that the pharmaceutically acceptable carrier be one, which has no detrimental side effects or toxicity under the conditions of use.

As used herein, the term "subject" refers to a human or another mammal (e.g., primate, dog, cat, goat, horse, pig, mouse, rat, rabbit, and the like), that can be the host of Hepatitis C virus (HCV), but may or may not be infected with the virus, and may or may not suffer from a HCV-related disease. Non-human subjects may be transgenic or otherwise modified animals. In many embodiments of the present invention, the subject is a human being. In such embodiments, the subject is often referred to as an "individual'. The term "individual" does not denote a particular age, and thus encompasses newborns, children, teenagers, and adults. As used herein, the term "HCV" refers to any major HCV genotype, subtype, isolate and/or quasispecies. HCV genotypes include, but are not limited to, genotypes 1, 2, 3, 4, 5, and 6; HCV subtypes include, but are not limited to, subtypes Ia, Ib, 2a, 2b, 2c, 3a, 4a- f, 5 a and 6a. The terms "afflicted with HCV" and "infected with HCV are used herein interchangeably. When used in reference to a subject, they refer to a subject that has at least one cell which is infected by HCV. The term "HCV infection" refers to the introduction of HCV genetic information into a target cell, such as by fusion of the target cell membrane with HCV or an HCV envelope glycoprotein-positive cell. The terms "HCV-related disease" and "HCV-associated disease" are herein used interchangeably. They refer to any disease or disorder known or suspected to be associated with and/or directly or indirectly caused by HCV. HCV-related (or HCV- associated) diseases include, but are not limited to, a wide variety of liver diseases, such as subclinical carrier state of acute hepatitis, chronic hepatitis, cirrhosis, and hepatocellular carcinoma. The terms include symptoms and side effects of any HCV infection, including latent, persistent and sub-clinical infections, whether or not the infection is clinically apparent.

The term "treatment" is used herein to characterize a method or process that is aimed at (1) delaying or preventing the onset of a disease or condition (e.g., HCV infection or HCV-related disease); (2) slowing down or stopping the progression, aggravation, or deterioration of the symptoms of the disease or condition; (3) bringing about amelioration of the symptoms of the disease or condition; or (4) curing the disease or condition. A treatment may be administered prior to the onset of the disease or condition, for a prophylactic or preventing action. Alternatively or additionally, a treatment may be administered after initiation of the disease, for a therapeutic action.

In **a seventh aspect,** the invention pertains to a method of manufacturing a HCV specific antigen recognizing construct expressing cell line, comprising
- providing a suitable host cell,
- providing a genetic construct comprising a coding sequence encoding the ABP of the invention,
- introducing into said suitable host cell said genetic construct,
- expressing said genetic construct by said suitable host cell.

The method may further comprise a step of cell surface presentation of said antigen recognizing construct on said suitable host cell.

In other preferred embodiments, the genetic construct is an expression construct comprising a promoter sequence operably linked to said coding sequence.

Preferably, said antigen recognizing construct is of mammalian origin, preferably of human origin. The preferred suitable host cell for use in the method of the invention is a mammalian cell, such as a human cell, in particular a human T lymphocyte. T-cells for use in the invention are described in detail herein above.

Also encompassed by the invention are embodiments, wherein said antigen recognizing construct is a modified TCR, wherein said modification is the addition of functional domains, such as a label or a therapeutically active substance. Furthermore, encompassed are TCR having alternative domains, such as an alternative membrane anchor domain instead of the endogenous transmembrane region.

Desirably, the transfection system for introducing the genetic construct into said suitable host cell is a retroviral vector system. Such systems are well known to the skilled artisan.

Also comprised by the present invention is in one embodiment the additional method step of isolation and purification of the antigen recognizing construct from the cell and, optionally, the reconstitution of the translated antigen recognizing construct-fragments in a T-cell.

In an alternative aspect of the invention a T-cell is provided obtained or obtainable by a method for the manufacture of a T-cell receptor (TCR) producing cell line as described, which TCR is specific for HCV infected cells and has high avidity as described herein above. Such a T-cell is depending on the host cell used in the method of the invention, for example, a human or non-human T-cell, preferably a human TCR.

Another aspect of the invention further pertains to a method for detecting a HCV antigen, or a complex of MHC and the HCV antigenic peptide (protein epitope of the HCV associated protein), in a (biological) sample -such as one obtained from a subject or patient - comprising contacting the sample with an ABP specifically binding to said HCV peptide presented on an infected cell, or to the HCV peptide/MHC complex, and detecting the binding between said ABP and said HCV peptide, or to the HCV peptide/MHC complex. In some embodiments, the antigen recognizing construct or ABP is a TCR or antibody, or similar con-structs, or preferably the antigen recognizing construct according to the herein described invention. In some embodiments, the (biological) sample is a sample of a liver (such as one of those described elsewhere herein) for example a sample comprising possible infected cells of a subject, such as liver cells.

Also provided is a method of treating HCV infection in a subject in need thereof, comprising:
- isolating a cell from said subject;
- transforming the cell with at least one vector encoding an antigen recognizing construct or ABP of the present invention to produce a transformed cell;
- expanding the transformed cell to produce a plurality of transformed cells; and
- administering the plurality of transformed cells to said subject.

Also provided is a method of treating HCV infection in a subject in need thereof, comprising:
- isolating a cell from a healthy donor;
- Transforming the cell with a vector encoding an antigen recognizing construct of the present invention to produce a transformed cell;
- expanding the transformed cell to produce a plurality of transformed cells; and
- administering the plurality of transformed cells to said subject.

Also provided is a method of detecting a HCV infection in a biological sample comprising:
- contacting the biological sample with an ABP of the present description, wherein the biological sample preferably comprises host cells infected with HCV;
- detecting binding of the ABP to the biological sample.

In some embodiments, the method of detecting HCV infection is carried out in vitro, in vivo or in situ.

Also provided is a method of detecting the presence of a condition in a mammal. The method comprises (i) contacting a sample comprising one or more cells from the mammal with any of the inventive TCRs (and functional variants thereof), polypeptides, proteins, nucleic acids, recombinant expression vectors, host cells, populations of cells, antibodies, or antigen binding portions thereof, or pharmaceutical compositions described herein, thereby forming a complex, and detecting the complex, wherein detection of the complex is indicative of the presence of the condition in the mammal, wherein the condition is a HCV infection.

With respect to the inventive method of detecting a condition in a mammal, the sample of cells can be a sample comprising whole cells, lysates thereof, or a fraction of the whole cell lysates, e.g., a nuclear or cytoplasmic fraction, a whole protein fraction, or a nucleic acid fraction.

For purposes of the inventive detecting method, the contacting can take place in vitro or in vivo with respect to the mammal. Preferably, the contacting is in vitro.

Also, detection of the complex can occur through any number of ways known in the art. For instance, the inventive antigen recognizing constructs (and functional variants thereof), polypeptides, proteins, nucleic acids, recombinant expression vectors, host cells, populations of cells, or antibodies or TCRs, or antigen binding portions thereof, described herein, can be labeled with a detectable label such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles).

For purposes of the inventive methods, wherein host cells or populations of cells are administered, the cells can be cells that are allogeneic or autologous to the mammal. Preferably, the cells are autologous to the mammal.

In general, the invention provides a method for treating a subject suffering from a HCV infection comprising the administration of the antigen recognizing constructs, ABPs, nucleic acids, vectors, pharmaceutical compositions and/or host cell as disclosed by the present invention. Preferably the subject is a subject in need of such a treatment. The subject in preferred embodiments is a mammalian subject, preferably a human patient, suffering from a HCV positive disease.

In addition to the above, the present invention further pertains to the following itemized embodiments:
Item 1: An isolated antigen binding protein (ABP) which specifically binds to a Hepatitis C virus associated antigenic peptide, or a variant thereof, and wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 3, 8, 13, 18, 23, 28, 33, 38, 43, and 48.
Item 2: The isolated ABP of item 1, wherein said ABP is capable of specifically and/or selectively binding to an antigenic peptide derived from Hepatitis C Virus (HCV).
Item 3: The isolated ABP of item 1 or 2, wherein the ABP is an antibody, or an antigen binding derivative or fragment thereof, or, preferably, is a T cell receptor (TCR), or an antigen binding derivative or fragment thereof.
Item 4: The isolated ABP of any one of items 1 to 3, wherein said ABP binds to a human leucocyte antigen (HLA) presented antigenic peptide, wherein said HLA is preferably type A2, preferably wherein the ABP forms a specific complex with the MHC and the antigenic peptide.
Item 5: The isolated ABP of any one of items 1 to 4, wherein the ABP specifically and/or selectively binds to an epitope having the amino acid sequence selected from an MHC presented peptide of a HCV protein, preferably a Core Protein or NS3 protein, and most preferably a peptide consisting of SEQ ID NO: 51 to 56, preferably 55 or 56.
Item 6:The isolated ABP of any one of items 1 to 5, wherein the ABP is an α/β-TCR, or antigen binding derivative or fragment thereof, or the construct is a γ/δ-TCR, or antigen binding derivative or fragment thereof.
Item 7: The isolated ABP of any one of items 1 to 6, characterized in that the ABP is of human origin and specifically and/or selectively recognizes an HCV antigenic peptide.
Item 8: The isolated ABP of any one of items 1 to 7, wherein said ABP is capable of inducing an immune response in a subject, optionally wherein the immune response is characterized by an increase in interferon (IFN) γ levels.
Item 9:The isolated ABP of any one of items 1 to 8, comprising a TCR α or γ chain; and/or a TCR β or δ chain; wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 3, 13, 23, 33, and 43; and/or wherein the TCR β or δ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 8, 18, 28, 38, and 48.
Item 10: The isolated ABP of item 9, wherein the TCR α or γ chain further comprises a CDR1 having an amino acid sequence with at least 80% sequence identity to, or having no more than three or two, preferably no more than one amino acid substitution(s), deletion(s) or insertion(s) compared to, a sequence selected from SEQ ID Nos: 1, 11, 21, 31, and 41; and/or a CDR2 having an amino acid sequence with at least 80% sequence identity to, or having no more than three or two, preferably no more than one amino acid substitution(s), deletion(s) or insertion(s) compared to, a sequence selected from SEQ ID Nos: 2, 12, 22, 32, and 42.
Item 11: The isolated ABP of item 9 or 10, wherein the TCR β or δ chain further comprises a CDR1 having an amino acid sequence with at least 80% sequence identity to, or having no more than three or two, preferably no more than one amino acid substitution(s), deletion(s) or insertion(s) compared to, a sequence selected from SEQ ID Nos: 6, 16, 26, 36, and 46; and/or a CDR2 having an amino acid sequence with at least 80% sequence identity to, or having no more than three or two, preferably no more than one amino acid substitution(s), deletion(s) or insertion(s) compared to, a sequence selected from SEQ ID Nos: 7, 17, 27, 37, and 47.
Item 12: The isolated ABP of any one of items 1 to 11, comprising a TCR variable chain region having at least 80% sequence identity to, or having no more than 20, preferably no more than 15, more preferably no more than 10, 5, three or two, preferably no more than one amino acid substitution(s), deletion(s) or insertion(s) compared to, an amino acid sequence selected from SEQ ID Nos. 4, 9, 14, 19, 24, 29, 34, 39, 44 and 49.
Item 13: The isolated ABP of any one of 1 to 12, wherein the construct is humanized, chimerized and/or murinized.
Item 14: The isolated ABP of any one of items 1 to 13, comprising a binding fragment of a TCR, and wherein said binding fragment comprises CDR1 to CDR3, optionally selected from the CDR1 to CDR3 sequences having the amino acid sequences of SEQ ID Nos. 1, 2, 3, or 6, 7, 8 or 11, 12, 13, or 16, 17, 18 or 21, 22, 23, or 26, 27, 28 or 31, 32, 33, or 36, 37, 38, or 41, 42, 43, or 46, 47, 48, each CDR independently having no more than three or two, preferably no more than one amino acid substitution(s), deletion(s) or insertion(s) compared to these sequences.
Item 15: The isolated ABP of any one of items 1 to 14, wherein the construct is a TCR, or a fragment thereof, composed of at least one TCR α and one TCR β chain sequence, wherein said TCR α chain sequence comprises the CDR1 to CDR3 sequences having the amino acid sequences of SEQ ID NO: 1 to 3, and said TCR β chain sequence comprises the CDR1 to CDR3 sequences having the amino acid sequences of SEQ ID NO: 6 to 8; or wherein said TCR α chain sequence comprises the CDR1 to CDR3 sequences having the amino acid sequences of SEQ ID NO: 11 to 13, and said TCR β chain sequence comprises the CDR1 to CDR3 sequences having the amino acid sequences of SEQ ID NO: 16 to 18; or wherein said TCR α chain sequence comprises the CDR1 to CDR3 sequences having the amino acid sequences of SEQ ID NO: 21 to 23, and said TCR β chain sequence comprises the CDR1 to CDR3 sequences having the amino acid sequences of SEQ ID NO: 26 to 28; or wherein said TCR α chain sequence comprises the CDR1 to CDR3 sequences having the amino acid sequences of SEQ ID NO: 31 to 33, and said TCR β chain sequence comprises the CDR1 to CDR3 sequences having the amino acid sequences of SEQ ID NO: 36 to 38; or wherein said TCR α chain sequence comprises the CDR1 to CDR3 sequences having the amino acid sequences of SEQ ID NO: 41 to 43, and said TCR β chain sequence comprises the CDR1 to CDR3 sequences having the amino acid sequences of SEQ ID NO: 46 to 48; each CDR independently having no more than three or two, preferably no more than one amino acid substitution(s), deletion(s) or insertion(s) compared to these sequences.
Item 16: The isolated ABP of any one of items 1 to 15, wherein the ABP is a TCR, or an antigen-binding fragment or derivative thereof, comprising at least one TCR α and one TCR β chain sequence, wherein said TCR α chain sequence comprises a variable region sequence having the amino acid sequence of SEQ ID No. 4, and wherein said TCR β chain sequence comprises a variable region sequence having the amino acid sequence of SEQ ID No. 9; or wherein said TCR α chain sequence comprises a variable region sequence having the amino acid sequence of SEQ ID No. 14, and wherein said TCR β chain sequence comprises a variable region sequence having the amino acid sequence of SEQ ID No. 19; or wherein said TCR α chain sequence comprises a variable region sequence having the amino acid sequence of SEQ ID No. 24, and wherein said TCR β chain sequence comprises a variable region sequence having the amino acid sequence of SEQ ID No. 29; or wherein said TCR α chain sequence comprises a variable region sequence having the amino acid sequence of SEQ ID No. 34, and wherein said TCR β chain sequence comprises a variable region sequence having the amino acid sequence of SEQ ID No. 39; or wherein said TCR α chain sequence comprises a variable region sequence having the amino acid sequence of SEQ ID No. 44, and wherein said TCR β chain sequence comprises a variable region sequence having the amino acid sequence of SEQ ID No. 49; each variable domain sequence having no more than 20, preferably no more than 15, more preferably no more than 10, 5, three or two, preferably no more than one amino acid substitution(s), deletion(s) or insertion(s) compared to these sequences.
Item 17: The isolated ABP of any one of items 1 to 16, wherein the ABP is a TCR, or an antigen-binding fragment or derivative thereof, further comprising a TCR constant region, preferably a human TCR constant region sequence.
Item 18: An isolated nucleic acid encoding for an ABP of any one of items 1 to 17.
Item 19: The isolated nucleic acid of item 18, comprising a nucleic acid sequence having at least 80% sequence identity to, or having no more than 20, preferably no more than 15, more preferably no more than 10, 5, three or two, preferably no more than one nucleic acid substitution(s), deletion(s) or insertion(s) compared to, a nucleic acid sequence selected from SEQ ID Nos 5, 10, 15, 20, 25, 30, 35, 40, and 45.
Item 20: A recombinant vector comprising a nucleic acid of item 18 or 19.
Item 21: A recombinant host cell comprising an ABP of any one of items 1 to 17, or a nucleic acid of item 18 or 19, or a vector of item 20.
Item 22: The recombinant host cell of item 21, wherein the cell is a lymphocyte, preferably a T lymphocyte or T lymphocyte progenitor, more preferably a CD4 or, most preferably a CD8 positive T-cell.
Item 23: A pharmaceutical composition comprising the ABP of any one of items 1 to 17, or a nucleic acid of item 18 or 19, or a vector of item 20, or the host cell of item 21 or 22, and a pharmaceutical acceptable carrier, stabilizer and/or excipient; preferably, wherein the pharmaceutical composition comprises at least two different ABP of any one of items 1 to 17.
Item 24: A compound or composition for use in medicine, wherein the compound or composition is selected from the ABP of any one of items 1 to 17, or a nucleic acid of item 18 or 19, or a vector of item 20, or the host cell of item 21 or 22, or the pharmaceutical composition of item 23.
Item 25: The compound or composition for use of item 24, wherein the use is for a treatment of an acute or chronic virus infection, preferably wherein the infection is an infection with a Hepatitis virus, such as HCV.
Item 26: The compound or composition for use of item 25, wherein the treatment comprises immune therapy, preferably adoptive autologous or heterologous T-cell therapy.
Item 27: The compound or composition for use of item 26, wherein the T-cell therapy involves the use of cell comprising the ABP or nucleic acid of any one of the preceding items.
Item 28: A method of manufacturing a HCV specific antigen recognizing construct expressing cell line, comprising
   (a) providing a suitable host cell,
   (b) providing a genetic construct comprising a coding sequence encoding the ABP according to any of items 1 to 17,
   (c) introducing into said suitable host cell said genetic construct,
   (d) expressing said genetic construct by said suitable host cell.
Item 29: The method of item 28, further comprising cell surface presentation of said antigen recognizing construct.
Item 30: The method of item 28 or 29, wherein the genetic construct is an expression construct comprising a promoter sequence operably linked to said coding sequence.
Item 31: The method of any one of items 28 to 30, wherein said antigen recognizing construct is of mammalian origin, preferably of human origin.
Item 32: The method of any one of items 28 to 31, wherein said suitable host cell is a mammalian cell, optionally selected from a human cell or a human T lymphocyte.
Item 33: The method of any one of items 28 to 32, wherein said antigen recognizing construct is a modified TCR, wherein said modification comprises addition of a functional domain comprising a label, or an alternative domain comprising a membrane anchor domain.
Item 34: The method of item 33 wherein said antigen recognizing construct is an ABP, preferably is an alpha/beta TCR, gamma/delta TCR, or a TCR variant such as a single chain TCR (scTCR).
Item 35: The method of any one of items 28 to 34, wherein said genetic construct is introduced into said suitable host cell by retroviral transfection.
Item 36: The method of any one of items 28 to 35, further comprising the isolation and purification of the antigen recognizing construct from the suitable host cell and, optionally, reconstitution of the antigen recognizing construct in a T-cell.
Item 37: The method of any one of items 28 to 35, wherein the suitable host cell is a cell, such as a T-cell, obtained from a patient suffering from a HCV infection.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1****: The HCV polyprotein.** The HCV genome is a positive RNA strand of 9.6 kb length flanked by 5' and 3' UTRs that function as IRES sites. The translational product is a large polyprotein of around 3000 amino acids that is proteolytically cleaved into structural proteins (Core, E1 and E2), p7 and non-structural proteins (NS2, NS3, NS4A, NS4B, NS5A and NS5B).

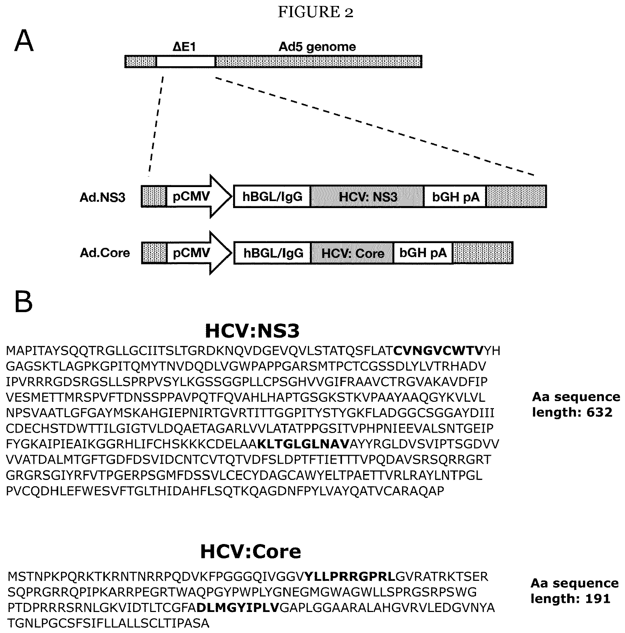

**Figure 2****: Recombinant adenoviruses encoding the HCV antigens NS3 and Core of the HCV genotype 1b polyprotein.** (**A**) The adenoviral plasmid constructs had been generated as described before (Iavoshian et al., Arch Virol, 2004, 149,:323) through deletion of the Ad5 genome region E3 and replacement of the E1 gene with a CMV immediate early enhancer/promoter (pCMV) driven expression cassette containing a chimeric human *β-*globulin/IgG intron (hBGL/IgG) followed by sequences derived from the HCV-J isolate genotype 1b corresponding to either the NS3 protein (Ad.NS3) or the Core protein (Ad.Core) and a bovine growth hormone polyadenylation signal (bGH pA). (**B**) Known HLA-A2 restricted epitopes within the NS3 (SEQ ID NO: 57) or Core (SEQ ID NO: 58) amino acid sequences are highlighted in bold.

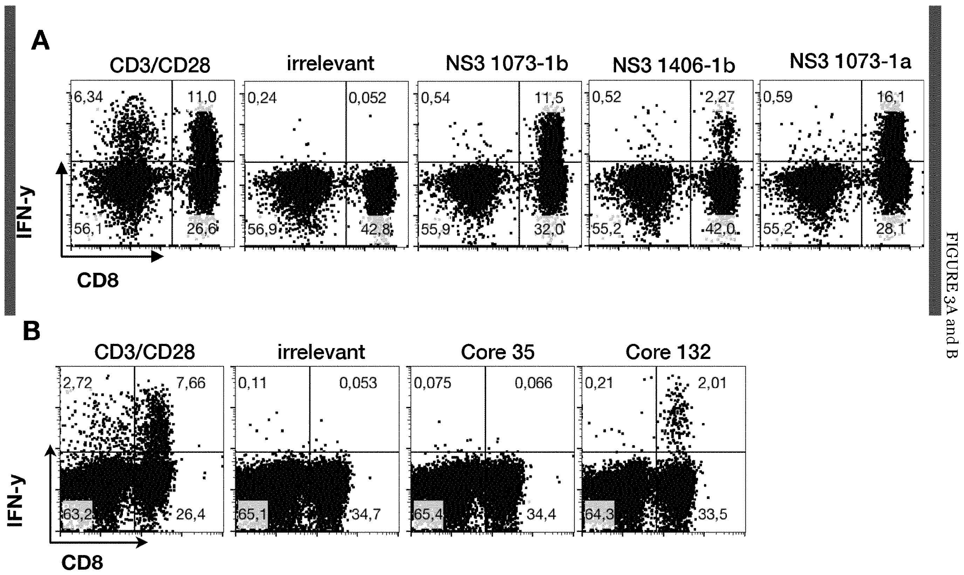

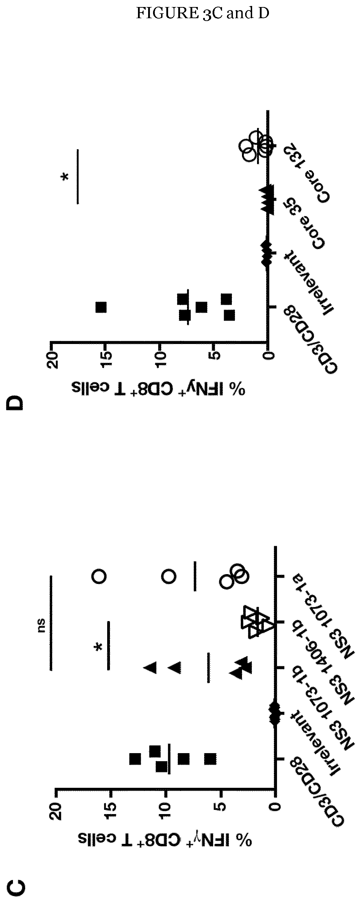

**Figure 3****: HCV epitopes NS3 1073-1b, NS3 1406-1b and Core 132 are immunogenic and naturally processed in ABabDII mice.** (**A-B**) Flowcytometry plots showing representative CD8⁺ T-cell responses of ABabDII mice immunized with the recombinant adenoviruses Ad.NS3-1b (A) or Ad.Core (B). Splenocytes were re-stimulated *ex vivo* with HCV peptide epitopes (as indicated) or NYESO peptide (irrelevant). Cells stimulated with Dynabeads (CD3/CD28) served as a positive control. In each row one representative mouse per group (n=5-6) is shown. (**C-D**) Dotplots summarizing the results of intracellular IFN-y stainings of all mice immunized with Ad.NS3-1b (C) or Ad.Core (D) showing the percentage of CD8⁺ IFN-y+ T-cells after specific peptide re-stimulation (as indicated).Taken together, it can be concluded that HLA-A2-restricted HCV epitopes NS3 1073-1b, NS3 1406-1b and Core 132 are immunogenic and naturally processed in ABabDII mice.

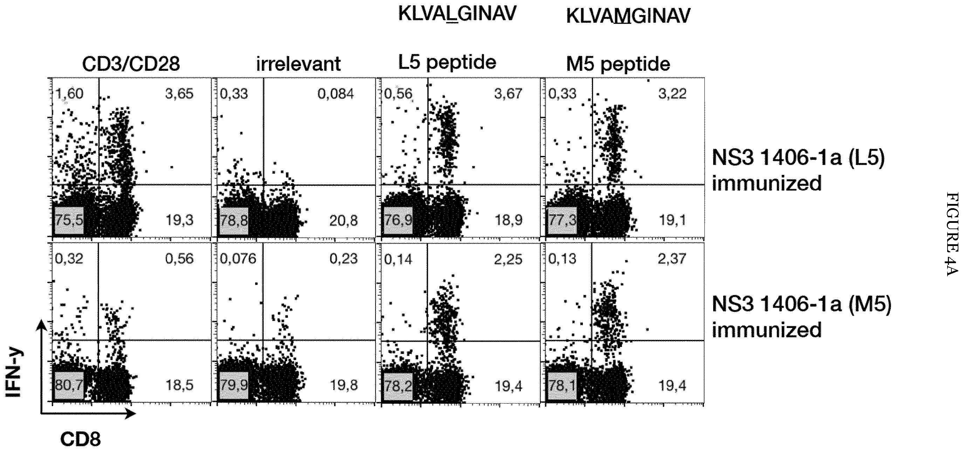

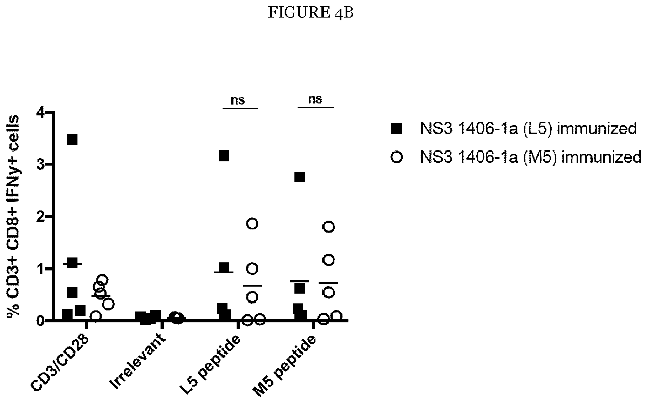

**Figure 4****: Epitopes NS3 1406-1a (L5) and (M5) are equally immunogenic in ABabDII mice and prime T-cells reacting to both epitopes**. (A) Flowcytometry plots showing representative CD8⁺ T-cell responses of mice immunized with the peptide epitope NS3 1406-1a (L5) or NS3 1406-1a (M5). Splenocytes were re-stimulated *ex vivo* with HCV peptide epitopes (as indicated) or peptide NY-ESO (irrelevant). Re-stimulation with Dynabeads (CD3/CD28) served as a positive control. In each row one representative mouse per group (n=5) is shown. (**B**) Dotplot summarizing the results of intracellular IFN-y stainings of all immunized mice showing the percentage of CD8⁺ IFN-y⁺ T-cells after specific peptide re-stimulation (as indicated).

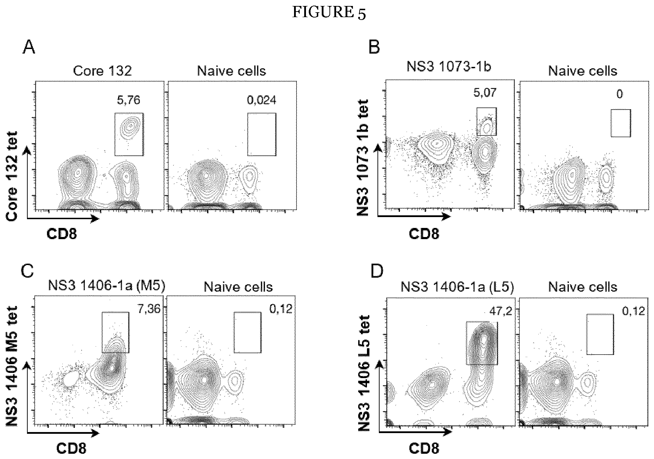

**Figure 5****: FACS sorts of A2-K^{b} HCV tetramer-labelled CD8⁺ T-cells from responder mice**. (**A-D**) Splenocytes from responder mice were cultured for 10 days in T-cell medium supplemented with 100 IU/ml IL-2 and 10⁻⁸ M specific HCV peptide epitope (as indicated). FACS plots show the FACS sorts of CD8⁺ A2-K^{b}tetramer labelled T-cells (pre-gated on living CD3⁺ cells). Cells in rectangular gates were sorted directly into lysis buffer for subsequent RNA isolation. Tetramer staining of splenocytes from naive ABabDII mice (naive cells) was used as a negative staining control.

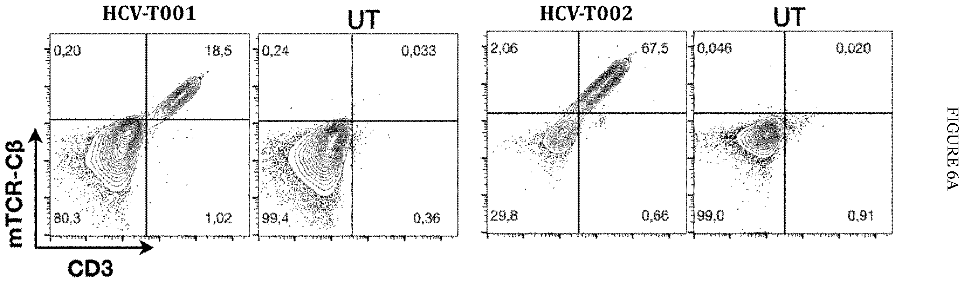

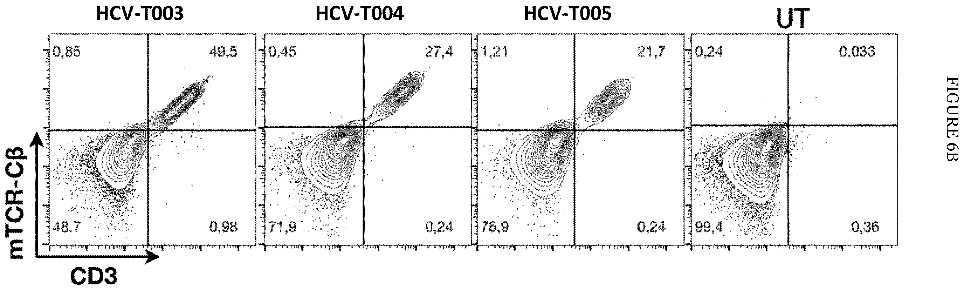

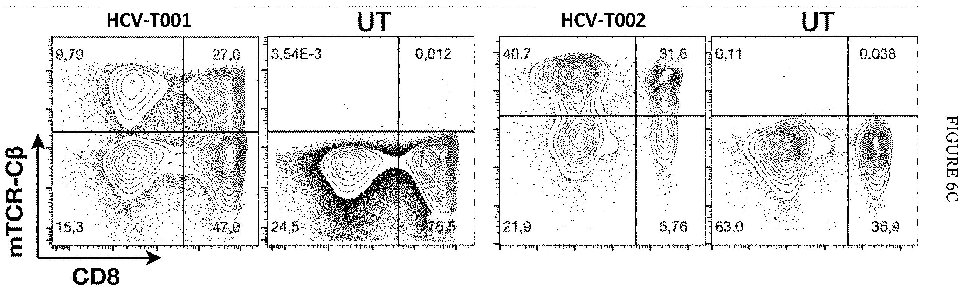

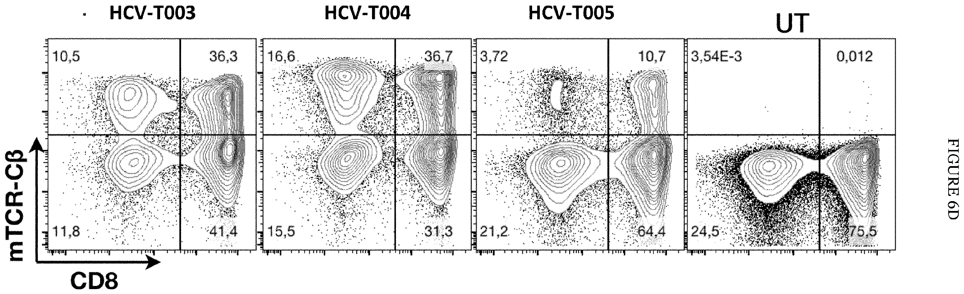

**Figure 6****: Re-expression of TCR constructs in human cells. (A-B)** FACS plots showing Jurkat cells that were transduced with either (A) HCV-T001 (left panel) or HCV-T002 (right panel) or (B) HCV-T003, HCV-T004 or HCV-T005 and stained with antibodies specific for the constant region of murine TCR-β (mTCR-Cβ) and human CD3⁺. Untransduced Jurkat cells (UT) were used as a negative control. **(C-D)** FACS plots showing human PBMCs that were transduced with either (C) HCV-T001 (left panel) or HCV-T002 (right panel) or (D) HCV-T003, HCV-T004 or HCV-T005 and stained with antibodies specific for the constant region of murine TCR-β (mTCR-Cβ) and human CD8⁺. Untransduced PBMCs of the same donor (UT) were used as a negative control. Cells were pre-gated on CD3⁺ lymphocytes. FACs plots are representative of stainings with n=3 different donor PBMCs. Dead cells were excluded through 7AAD staining.

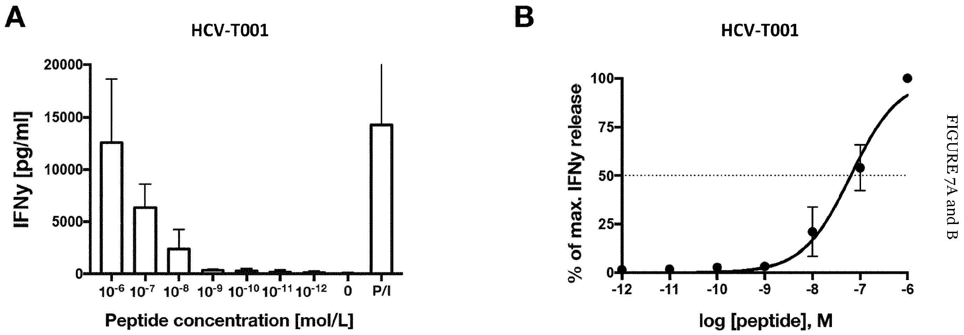

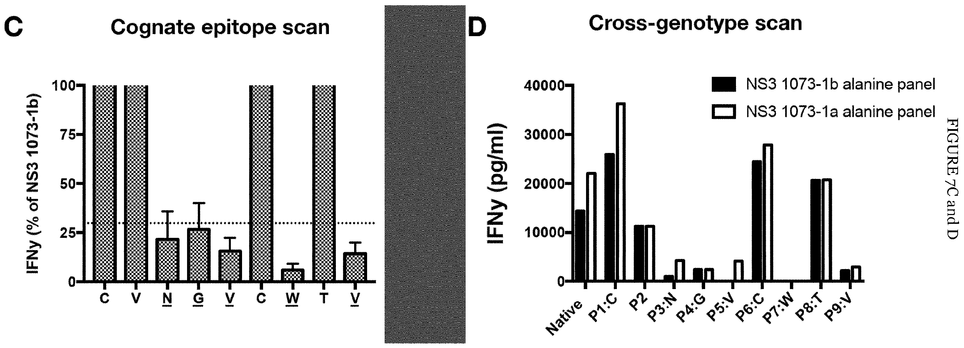

**Figure** 7: **HCV-T001 transduced PBMCs recognize the cognate epitope NS3 1073-1b and naturally occurring epitope variants.** (**A**) HCV-T001 transduced PBMC were co-cultured with T2 cells loaded with decreasing amounts of NS3 1073-1b peptide and IFN-y secretion was measured. Stimulation with PMA/Iono (P/I) served as a positive control. (**B**) Data from (A) fitted to a fourparameter logistic model normalized to maximum IFN-y release. (**C**) Amino acid positions of epitope NS3 1073-1b were exchanged with alanine, loaded on T2 cells at a concentration of 10⁻⁶ M and cocultured with HCV-T001 transduced PBMCs. IFN-y secretion in response to unmodified NS3 1073-1b peptide was defined as 100%. Underlined is the HCV-T001 recognition motif. Experiments (A-C) were done with PBMCs from three different healthy donors. (**D**) HCV-T001 transduced PBMCs were co-cultured with T2 cells loaded with cognate peptide NS3 1073-1b or NS3 1073-1a (native) or alanine scan panels based on both epitopes. Shown is data from one experiment with HCV-T001 transduced PBMC from the same donor.

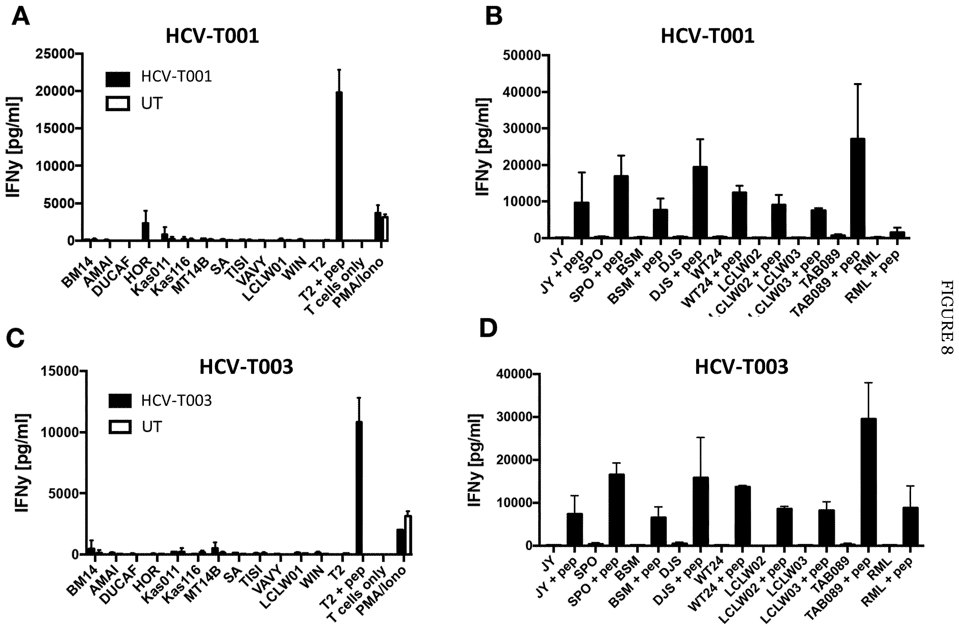

**Figure 8****: HCV-T001 or HCV-T003 transduced PBMC were co-cultured with a panel of B-LCLs expressing different HLA molecules.** (**A**) PBMCs transduced with HCV-T001 or untransduced PBMCs (UT) were co-cultured with a panel of B-LCL cell lines expressing different HLA molecules and IFN-y secretion was measured. T2 cells loaded with 10⁻⁶M cognate peptide epitope (+ pep) and restimulation with PMA/Iono served as positive control. (**B**) PBMCs transduced with HCV-T001 were co-cultured with a panel of HLA-A*02:01 positive B-LCL cell lines that were either left untreated or loaded with 10⁻⁶ M cognate peptide epitope (+ pep). All graphs (A-B) show data from two independent experiments using PBMCs from different donors.

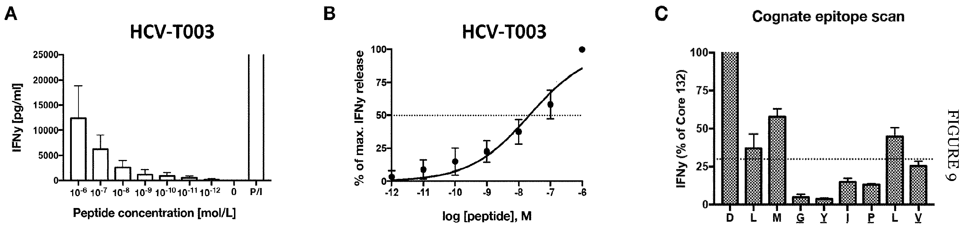

**Figure 9****: Characterization of HCV-T003.** (**A**) Human PBMCs transduced with **HCV-T003** were co-cultured with T2 cells loaded with decreasing amounts of Core 132 peptide and secreted IFN-y was measured. Stimulation with PMA/Iono (P/I) served as a positive control. (**B**) Plot showing the data from (A) fitted to a four-parameter logistic model normalized to maximum IFN-y release. (**C**) Individual amino acid positions of peptide Core 132 were exchanged with alanine, loaded on T2 cells at a concentration of 10⁻⁵ M and co-cultured with HCV-T003 transduced PBMCs. The IFN-y secretion in response to unmodified Core 132 peptide was defined as 100%. Underlined is the HCV-T003 recognition motif. Shown is data from two independent experiments with two different donor PBMCs.

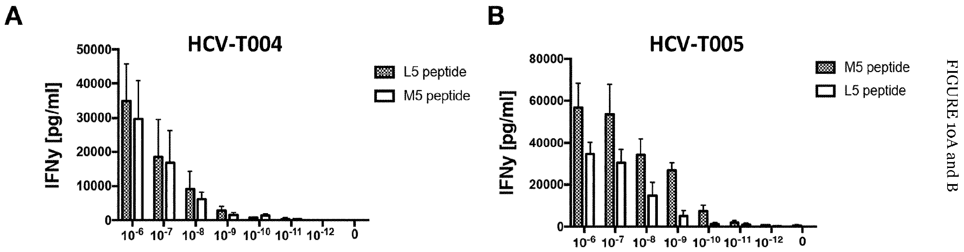

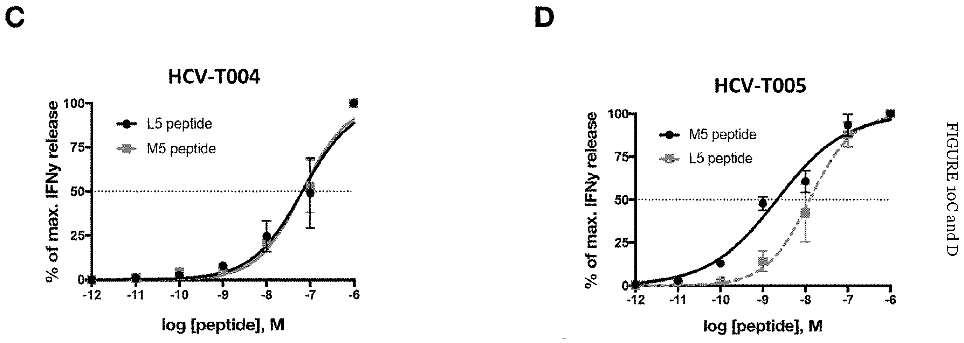

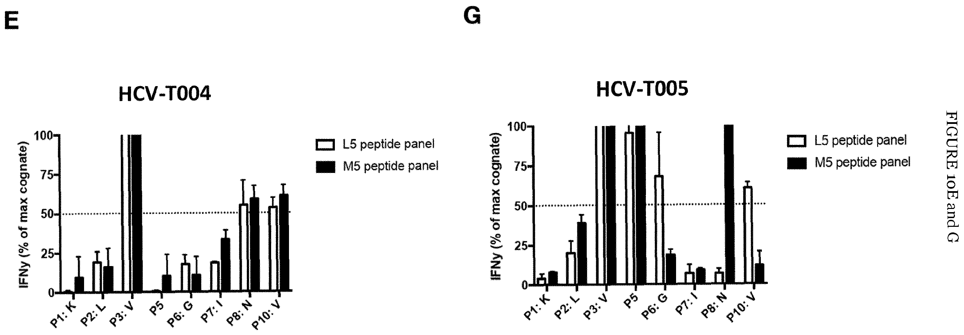

**Figure 10****: Characterization of HCV-T004 and HCV-T005.** PBMCs were transduced with HCV-T004 or HCV-T005 and co-cultured with peptide loaded T2 cells and IFN-y secretion was measured. (**A-B**) HCV-T005 or HCV-T004 transduced PBMCs were stimulated with T2 cells and decreasing amounts of L5 or M5 peptide. Shown is data from three independent experiments using different donor PBMCs. (**C-D**) Data from (A) or (B) was fitted to a four-parameter logistic model normalized to maximum IFN-y release. (E,G) HCV-T004 or HCV-T005 transduced PBMCs were co-cultured with an alanine scan panel based on either peptide L5 or M5. T2 cells were loaded with 10⁻⁶ M (E) or with 10⁻⁵ M peptide (G). Both plots show data from two independent experiments with different donor PBMCs.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Selection and in silico analysis of HCV-derived CD8+ T-cell epitopes

For immunization experiments, two recombinant adenoviral vectors were available coding for full NS₃ and Core protein sequences that comprise the HLA-A2 restricted epitopes NS3 1073-1b (CVNGVCWTV) and NS3 1406-1b (KLTGLGLNAV) as well as the epitopes Core 35 (YLLPRRGPRL) and Core 132 (DLMGYIPLV), respectively (Figure 2). Literature research on these epitopes revealed that all of them had previously been described in naturally occurring CD8+ T-cell responses in HCV-infected humans and/or as being immunogenic in HLA-A2 transgenic mice that are deficient for murine H-2Db and β2m expression (HHD mice) (Pascolo et al. 1997) (Table A). However, the epitope NS31406-1b has so far only been described to be immunogenic in HHD mice and is overall less conserved compared to other HCV epitopes (Himoudi et al. 2002). In addition, the immunodominant epitope NS3 1406-1a (L5) (KLVALGINAV) and the epitope escape variant NS3 1406-1a (M5) (KLVAMGINAV) were included in the analysis in order to test the hypothesis of HCV escaping de novo T-cell recognition through a "hole in the T-cell repertoire" (Wölfl et al. 2008). Since no recombinant adenoviral vectors encoding these two epitopes were available, it was decided to test their immunogenicity through short peptide immunization.

**Table A Predicted binding affinity of HCV epitopes to HLA-A2 and their immunogenicity in humans and HHD mice.**

| | | | | | **Immunogenicity†** | |
|---|---|---|---|---|---|---|
| **HCV epitope** | **Sequence** | **SEQ ID NO:** | **Length** | **Predicted IC50 (nM)*** | **Humans** | **HHD mice** |
| **Core 35** | YLLPRRGPRL | 51 | 10mer | 245,22 | [5] | [2] |
| **Core 132** | DLMGYIPLV | 52 | 9mer | 9,08 | [6] | [2] |
| **NS3 1073-1b** | CVNGVCWTV | 53 | 9mer | 268,23 | [4] | [2] |
| **NS3 1406-1b** | KLTGLGLNAV | 54 | 10mer | 67,74 | - | [2] |
| **NS3 1406-1a L5** | KLVALGINAV | 55 | 10mer | 40,72 | [4] | [1] |
| **NS3 1406-1a M5** | KLVAMGINAV | 56 | 10mer | 36,68 | [3] | - |

| | | | | | | |
|---|---|---|---|---|---|---|
| * predicted using NetMHC version 4.0 (DTU Bioinformatics) accessed in July 2018 t indicated are publications reporting CD8+ T-cell responses for the respective epitope in HCV infected humans (Humans) or HLA.A2 transgenic mice immunized with HCV antigen (HHD mice) | | | | | | |

One of the factors determining the immunogenicity of a T-cell epitope is its binding efficiency to MHC class I molecules and those epitopes exceeding an affinity threshold of 500 nM are unlikely to elicit successful T-cell responses (Sette et al. 1994). In silico analysis showed that all selected HCV epitopes exhibited predicted binding affinities below 500 nM (Table A). Taken together, the selected HCV epitopes were hypothesized to be immunogenic in ABabDII mice based on their predicted HLA-A2 binding affinities and their reported immunogenicity in HCV-infected humans and/or immunized HHD mice.

ABabDII mice were immunized either with recombinant adenoviruses encoding for full HCV protein sequences (Ad.NS3 or Ad.Core) or with the short peptide 10-mer epitope sequences KLVALGINAV or KLVAMGINAV. CD8+ T-cell responses were then measured ex vivo through restimulation of splenocytes with specific HCV peptide epitopes and intracellular staining for murine IFN-y. The magnitude of CD8+ T-cell responses was quantified as the percentage of CD8 and IFN-y positive cells.

Immunization with recombinant adenovirus Ad.NS3 elicited robust CD8+ T-cell responses to both epitopes NS3 1073-1b and NS3 1406-1b in all immunized mice (n=5), but not to irrelevant peptide. The responses to NS3 1073-1b were thereby on average 3.7 fold higher (P = 0.0412, t-test) than the ones to NS3 1406-1b suggesting an immunodominance pattern (Figure 3 A,C). Of note, also re-stimulation with the HCV genotype 1a derived epitope variant NS3 1073-1a (CINGVCWTV), which differs in only one amino acid from epitope NS3 1073-1b, elicited CD8+ T-cell responses of identical magnitude (P = 0.6871, t-test) indicating cross-genotype reactivity (Figure 3A,C). Immunization with recombinant adenovirus Ad.Core, however, generated CD8+ T-cell responses to epitope Core 132 in only half of the immunized mice (n=3/6), but no detectable response to epitope Core 35 or irrelevant peptide (Figure 3B,D). The responses to Core 132 were significantly higher than to Core 35 (difference: P = 0.0435, t-test) suggesting also an immunodominance pattern (Figure 3B,D). Taken together, it can be concluded that HLA-A2-restricted HCV epitopes NS3 1073-1b, NS3 1406-1b and Core 132 are immunogenic and naturally processed in ABabDII mice.

In order to test the hypothesis of HCV escaping CD8+ T-cell recognition through exploiting a "hole in the T-cell repertoire" (Wölfl et al. 2008), mice were immunized with short peptide representing either the original NS3 1406-1a (L5) epitope (KLVALGINAV) or the epitope escape variant NS3 1406-1a (M5) (KLVAMGINAV). It was found that immunization with L5 peptide primed CD8+ T-cells responding equally well to L5 or M5 re-stimulation with the same magnitude of IFN-y secretion (P = 0.8094, t-test) while not responding to re-stimulation with irrelevant peptide. Similarly, M5 immunized mice harbor CD8+ T-cells that respond equally to M5 or L5 re-stimulation (P = 0.5136, t-test) and did not react to irrelevant peptide re-stimulation (Figure 4A,B). Taken together, it was found that epitopes NS3 1406-1a (L5) and NS3 1406-1a (M5) are both immunogenic in ABabDII mice provoking CD8+ T-cell responses of identical magnitude that show reactivity to both peptide epitopes.

### Example 2: Isolation and identification of HCV-specific TCRs

The HCV epitopes Core 132, NS3 1073-1b, NS3 1406-1a (L5) and NS3 1406-1a (M5) were selected for human TCR gene isolation. To this end, cultured splenocytes from responder mice were labelled with HCV epitope specific A2-Kb tetramers as well as antibodies for murine CD3 and CD8 and FACS sorted into lysis buffer (Figure 5A-D). The number of CD8+ T-cells per sorted sample varied between 800 and 50.000 cells (Figure 5E). Next RNA was extracted from lysed cells and transcribed into cDNA for carrying out TCRα or TCRβ chain-specific 5'-RACE PCR reactions. DNA from each reaction was sequenced and the rearranged TCRα and TCRβ genes were identified. It was found that the clonality of TCRα and TCRβ chains ranged mostly between 80 and 100% indicating clonal CD8+ T-cell expansion in all samples (Table 1 and 2). However, the material obtained from cells labelled with NS3 1073-1b-A2-Kb tetramer exhibited less clonality regarding the TCRα chain usage, since two possible TCRα chains (TRAV14*01 and TRAV22*01) and one possible TCRβ chain (TRBV28*01) were detected.

**Table 1: Isolated TCR Sequences of the invention**

| **TCR Designation** | **TCR Chain** | **TCR Domain** | **Sequence** | **SEQ ID NO:** |
|---|---|---|---|---|
| HCV-T001 | alpha | CDRA1 | TSDPSYG | 1 |
| HCV-T001 | alpha | CDRA2 | QGSYDQQN | 2 |
| HCV-T001 | alpha | CDRA3 | AMREYSGGSNYKLT | 3 |
| HCV-T001 | alpha | Variable Domain A(protein) V-J Region | AQKITQTQPGMFVQEKEAVTLDCTYDTS DPSYGLFWYKQPSSGEMIFLIYQGSYDQ QNATEGRYSLNFQKARKSANLVISASQL GDSAMYFCAMREYSGGSNYKLTFGKGT LLTVNP | 4 |
| HCV-T001 | alpha | Variable Domain A (nucleic acid) V-J Region | gcccagaagatcacccagacccagcccggcatgttcgtgc aggaaaaagaagccgtgacc ctggactgcacctacgacaccagcgaccctagctacggac tgttttggtacaagcagccc agcagcggcgagatgatcttcctgatctaccagggctccta cgaccagcagaacgccacc gagggccggtacagcctgaacttccagaaggcccggaag tccgccaacctcgtgatcagc gctagccagctgggcgacagcgccatgtacttttgcgccat gagagagtacagcggcggc tccaactacaagctgaccttcggcaagggcacactgctga ccgtgaacccca | 5 |
| HCV-T001 | beta | CDRB1 | MDHEN | 6 |
| HCV-T001 | beta | CDRB2 | SYDVKM | 7 |
| HCV-T001 | beta | CDRB3 | ASSLLLAGVGETQY | 8 |
| HCV-T001 | beta | Variable Domain B (protein) V-J Region | DVKVTQSSRYLVKRTGEKVFLECVQDM DHENMFWYRQDPGLGLRLIYFSYDVKM KEKGDI PEGYSVSREKKERFSLILESASTNQTSMY LCASSLLLAGVGETQYFGPGTRLLVL | 9 |
| HCV-T001 | beta | Variable Domain B (nucleic acid) V-J Region | gacgtgaaagtgacccagagcagcagatacctcgtgaag cggaccggcgagaaggtgttc ctggaatgcgtgcaggacatggaccacgagaatatgttct ggtacagacaggaccccggc ctgggcctgcggctgatctacttcagctacgacgtgaagat gaaggaaaagggcgacatc cccgagggctacagcgtgtccagagagaagaaagagcg gttcagcctgatcctggaaagc gccagcaccaaccagaccagcatgtacctgtgcgccagct ccctgctgctggctggcgtg ggagagacacagtactttggccctggcaccagactgctgg tgctgg | 10 |
| HCV-T002 | alpha | CDRA1 | DSVNN | 11 |
| HCV-T002 | alpha | CDRA2 | IPSGT | 12 |
| HCV-T002 | alpha | CDRA3 | AVEPNSGNTPLV | 13 |
| HCV-T002 | alpha | Variable Domain A(protein) V-J Region | GIQVEQSPPDLILQEGANSTLRCNFSDSV NNLQWFHQNPWGQLINLFYIPSGTKQN GRLS ATTVATERYSLLYISSSQTTDSGVYFCAV EPNSGNTPLVFGKGTRLSVIA | 14 |
| HCV-T002 | alpha | Variable Domain A (nucleic acid) V-J Region | ggaatacaagtggagcagagtcctccagacctgattctcc aggagggagccaattccacg ctgcggtgcaatttttctgactctgtgaacaatttgcagtgg tttcatcaaaacccttgg ggacagctcatcaacctgttttacattccctcagggacaaa acagaatggaagattaagc gccacgactgtcgctacggaacgctacagcttattgtacat ttcctcttcccagaccaca gactcaggcgtttatttctgtgctgtggagccgaattcagg aaacacacctcttgtcttt ggaaagggcacaagactttctgtgattgcaa | 15 |
| HCV-T002 | beta | CDRB1 | MDHEN | 16 |
| HCV-T002 | beta | CDRB2 | SYDVKM | 17 |
| HCV-T002 | beta | CDRB3 | ASSLLLAGVGETQY | 18 |
| HCV-T002 | beta | Variable Domain B (protein) | DVKVTQSSRYLVKRTGEKVFLECVQDM DHENMFWYRQDPGLGLRLIYFSYDVKM KEKGDI PEGYSVSREKKERFSLILESASTNQTSMY LCASSLLLAGVGETQYFGPGTRLLVL | 19 |
| HCV-T002 | beta | Variable Domain B (nucleic acid) | gacgtgaaagtgacccagagcagcagatacctcgtgaag cggaccggcgagaaggtgttc ctggaatgcgtgcaggacatggaccacgagaatatgttct ggtacagacaggaccccggc ctgggcctgcggctgatctacttcagctacgacgtgaagat gaaggaaaagggcgacatc cccgagggctacagcgtgtccagagagaagaaagagcg gttcagcctgatcctggaaagc gccagcaccaaccagaccagcatgtacctgtgcgccagct ccctgctgctggctggcgtg ggagagacacagtactttggccctggcaccagactgctgg tgctgg | 20 |
| HCV-T003 | alpha | CDRA1 | TSWWSY | 21 |
| HCV-T003 | alpha | CDRA2 | RQGSDEQN | 22 |
| HCV-T003 | alpha | CDRA3 | ALGENSGYSTLT | 23 |
| HCV-T003 | alpha | Variable Domain A(protein) V-J Region | AQKVTQAQSSVSMPVRKAVTLNCLYETS WWSYYIFWYKQLPSKEMIFLIRQGSDEQ NAKS GRYSVNFKKAAKSVALTISALQLEDSAKY FCALGENSGYSTLTFGKGTMLLVSP | 24 |
| HCV-T003 | alpha | Variable Domain A (nucleic acid) V-J Region | gcccagaaagtgacacaggcccagtcctccgtgtccatgc ctgtgcggaaagccgtgacc ctgaactgcctgtacgaaacctcctggtggtcctactacatc ttctggtacaagcagctg cccagcaaagagatgatcttcctgatccggcagggcagcg acgagcagaatgccaagagc ggccggtacagcgtgaacttcaagaaagccgccaagtcc gtggccctgaccatctctgct ctgcagctggaagatagcgccaagtacttctgtgccctggg cgagaactccggctactcc accctgacctttggcaagggcaccatgctgctggtgtcccc ca | 25 |
| HCV-T003 | beta | CDRB1 | KGHDR | 26 |
| HCV-T003 | beta | CDRB2 | SFDVKD | 27 |
| HCV-T003 | beta | CDRB3 | ATSDLGLAGYNEQF | 28 |
| HCV-T003 | beta | Variable Domain B (protein) V-J Region | DADVTQTPRNRITKTGKRIMLECSQTKG HDRMYWYRQDPGLGLRLIYYSFDVKDI NKGEI SDGYSVSRQAQAKFSLSLESAIPNQTALY FCATSDLGLAGYNEQFFGPGTRLTVL | 29 |
| HCV-T003 | beta | Variable Domain B (nucleic acid) V-J Region | gacgccgatgtgacccagacccccggaacagaatcacc aagaccggcaagcggatcatg ctggaatgcagccagaccaagggccacgaccggatgtac tggtacagacaggaccctggc ctgggcctgcggctgatctactacagcttcgacgtgaagga catcaacaagggcgagatc agcgacggctacagcgtgtccagacaggctcaggccaag ttcagcctgtccctggaaagc gccatccccaaccagaccgccctgtacttctgcgccacaa gcgatctgggcctggccggc tacaacgagcagttctttggccctggcacccggctgaccgt gctgg | 30 |
| HCV-T004 | alpha | CDRA1 | TSESDYY | 31 |
| HCV-T004 | alpha | CDRA2 | QEAYKQQN | 32 |
| HCV-T004 | alpha | CDRA3 | AYRDDKII | 33 |
| HCV-T004 | alpha | Variable Domain A(protein) V-J Region | AQTVTQSQPEMSVQEAETVTLSCTYDTS ESDYYLFWYKQPPSRQMILVIRQEAYKQ QNAT ENRFSVNFQKAAKSFSLKISDSQLGDAA MYFCAYRDDKIIFGKGTRLHILP | 34 |
| HCV-T004 | alpha | Variable Domain A (nucleic acid) V-J Region | gcccagaccgtgacacagagccagcccgagatgagcgtg caggaagccgagacagtgacc ctgagctgcacctacgacaccagcgagtccgactactacc tgttctggtacaagcagccc cccagccggcagatgatcctcgtgatcagacaggaagcct acaagcagcagaacgccacc gagaacagattcagcgtgaacttccagaaggccgccaag agctttagcctgaagatcagc gacagccagctgggcgacgccgccatgtacttttgcgccta ccgggacgataagatcatc ttcggcaagggcacccggctgcacatcctgccca | 35 |
| HCV-T004 | beta | CDRB1 | MGHDK | 36 |
| HCV-T004 | beta | CDRB2 | SYGVNS | 37 |
| HCV-T004 | beta | CDRB3 | ASSDGDTQY | 38 |
| HCV-T004 | beta | Variable Domain B (protein) V-J Region | EADIYQTPRYLVIGTGKKITLECSQTMG HDKMYWYQQDPGMELHLIHYSYGVNS TEKGDL SSESTVSRIRTEHFPLTLESARPSHTSQY LCASSDGDTQYFGPGTRLTVL | 39 |
| HCV-T004 | beta | Variable Domain B (nucleic acid) V-J Region | gaagccgacatctaccagacccccagatacctcgtgatcg gcaccggcaagaaaatcacc ctggaatgcagccagaccatgggccacgacaagatgtact ggtatcagcaggaccccggc atggaactgcacctgatccactacagctacggcgtgaaca gcaccgagaagggcgacctg agcagcgagagcaccgtgtccagaatccggaccgagcac ttcccactgaccctggaaagc gctagacccagccacaccagccagtacctgtgtgccagca gcgacggcgacacccagtat tttggccctggcaccagactgaccgtgctgg | 40 |
| HCV-T005 | alpha | CDRA1 | TSESDYY | 41 |
| HCV-T005 | alpha | CDRA2 | QEAYKQQN | 42 |
| HCV-T005 | alpha | CDRA3 | AYSEYGNKLV | 43 |
| HCV-T005 | alpha | Variable Domain A(protein) V-J Region | AQTVTQSQPEMSVQEAETVTLSCTYDTS ESDYYLFWYKQPPSRQMILVIRQEAYKQ QNAT ENRFSVNFQKAAKSFSLKISDSQLGDAA MYFCAYSEYGNKLVFGAGTILRVKS | 44 |
| HCV-T005 | alpha | Variable Domain A (nucleic acid) V-J Region | gcccagaccgtgactcagagccagcccgagatgagcgtg caggaagccgagacagtgacc ctgagctgcacctacgacaccagcgagagcgactactacc tgttctggtacaagcagccc cccagccggcagatgatcctcgtgattagacaggaagcct ataagcagcagaacgccaca gagaacagattcagcgtgaacttccagaaggccgccaag agctttagcctgaagatcagc gacagccagctgggcgacgccgccatgtatttctgcgccta cagcgagtacggcaacaag ctggtgttcggagccggcacaatcctgagagtgaagtcct | 45 |
| HCV-T005 | beta | CDRB1 | MNHEY | 46 |
| HCV-T005 | beta | CDRB2 | SMNVEV | 47 |
| HCV-T005 | beta | CDRB3 | ASSLFRSSYNEQF | 48 |
| HCV-T005 | beta | Variable Domain B (protein) V-J Region | EAQVTQNPRYLITVTGKKLTVTCSQNMN HEYMSWYRQDPGLGLRQIYYSMNVEVT DKGDV PEGYKVSRKEKRNFPLILESPSPNQTSLY FCASSLFRSSYNEQFFGPGTRLTVL | 49 |
| HCV-T005 | beta | Variable Domain B (nucleic acid) V-J Region | gaagcccaagtgacccagaaccccagatacctgatcacc gtgaccggcaagaaactgacc gtgacctgcagccagaacatgaaccacgagtacatgagct ggtacagacaggaccccggc ctgggcctgcggcagatctactacagcatgaacgtggaag tgaccgacaagggcgacgtg cccgagggctacaaggtgtcccggaaagagaagcggaac ttcccactgatcctggaaagc cccagccccaaccagaccagcctgtacttctgtgccagca gcctgttcagaagcagctac aacgagcagttcttcggccctggcacccggctgaccgtgct gg | 50 |

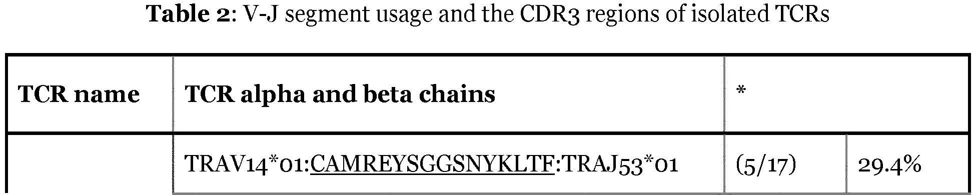

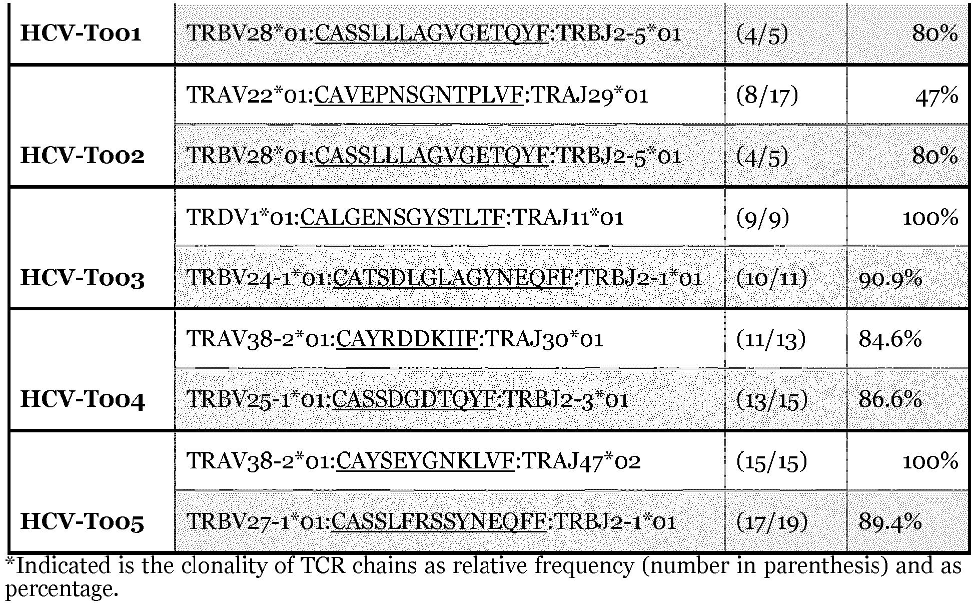

### Example 3: HCV-specific TCRs are re-expressed in human cells

The most frequent TCRα and TCRβ chains per sort were combined and TCR expression cassettes were constructed connecting the TCRβ chain with the TCRα chain through a P2A linker element derived from the porcine teschovirus (Leisegang et al. 2008). For a later clinical application and optimal TCR expression in human cells, all sequences were optimized for human codon usage (Scholten et al. 2006). In addition, the constant regions of the human TCRα and TCRβ chains were fully murinized through replacing the human nucleotide sequences with murine constant region sequences enabling a preferential pairing of murine constant regions and to avoid TCR chain mis-pairing (Cohen et al. 2006). Since for the 1073-1b specific TCR two TCRα chains were detected, two TCR constructs (HCV-T001 and TCR 73-2) were generated in order to test both combinations in further experiments. All TCR expression cassettes were cloned into the retroviral expression vector pMP71. Transduction of TCR negative Jurkat 76 cells with TCR constructs led to robust expression of all TCRs indicating the ability of the re-expressed TCR chains to pair and form stable TCR/CD3 complexes at the cell surface (Figure 6A,B). Similarly, also transduction of human PBMCs from healthy donors resulted in robust TCR cell surface expression of all TCRs in three different donors (Figure 6C,D). Of note, re-staining of TCR transduced cells with the respective HCV-specific A2-Kb tetramer used for TCR isolation led to significant background staining on Jurkat cells as well as PBMCs (data not shown) and was therefore discarded as a tool for validating TCR re-expression. Taken together it can be concluded that all TCR constructs are transferrable to different human target cells and are stably re-expressed on the cell surface.

### Example 4: Characterization of HCV-specific TCRs

The reactivity of each TCR was analyzed through co-culturing TCR-transduced PBMCs with peptide-pulsed T2 cells and measurement of IFN-y secretion. The functional avidity of each TCR was determined through peptide titration experiments loading decreasing amounts of cognate peptide epitope on T2 cells. In addition, alanine scan experiments were performed loading T2 cells with peptides that were mutated to express the amino acid alanine at each position of the cognate peptide epitope. A threshold of 30% of the maximal IFN-y secretion was set to determine a TCR recognition motif that is minimally needed for TCR activation. The TCR recognition motifs were analyzed using the database Expasy Prosite to screen for peptides with the same motif within the human proteome. Moreover, approximately 10% of naive T-cell precursors in the human T-cell repertoire are allo-reactive (Sherman et al. 1993) and since ABabDII mouse-derived human T-cells human are selected on only one human HLA molecule, the possibility of allo-HLA reactivity of isolated TCRs has to be tested in order to avoid off-target toxicity in ATT. Therefore, HCV- specific TCR-transduced PBMCs were co-cultured with a panel of EBV transformed B-lymphoblastoid cell lines (B-LCLs) expressing different HLA molecules.

TCR 73-1 or TCR 73-2 transduced PBMCs were co-cultured with T2 cells pulsed with decreasing amounts of epitope NS3 1073-1b. It was found that TCR 73-1 transduced cells secreted robust amounts of IFN-y (2400 pg/ml) up until a concentration of 10-8 M stimulating peptide. The functional avidity of TCR 73-1 was determined to be EC50 = 6.42x10-8 M (Figure 7 A,B). cognate peptide and this TCR was therefore discarded (data not shown). Alanine scan experiments with TCR 73-1 transduced PBMCs showed that amino acid positions 3,4,5,7 and 9 of epitope NS3 1073-1b (CVNGVCWTV) were minimally required for recognition revealing the TCR 73-1 recognition motif (xxNGVxWxV) (Figure 7C). Search in the human proteome revealed only one human peptide with the same motif derived from the human Zinc finger protein (EENGVRWHV). The predicted binding affinity of this peptide to HLA-A2 molecules, however, was found to be very low (25961.22 nM) making it unlikely to function as a CD8+ T cell epitope. However, a range of NS3 1073 derived peptides was found in silico in different isolates of HCV strains of genotypes 1a, 1b, 4a, 5a, 6a, 6b, 6d, 6g, 6h and 6k that also contain the TCR 73-1 recognition motif. A cross-genotype alanine scan was done using an alanine panel based on NS3 1073-1b or NS3 1073-1a in parallel (Appendix 3), which confirmed that TCR 73-1 cross-reacts to epitope NS3 1073-1a (CINGVCWTV) and shows that TCR-73-1 uses the same pattern for NS3 1073-1b and NS3 1073-1a recognition (Figure 7D). Several NS3 RASs had been reported to emerge in HCV-infected patients after treatment with NS3 protease inhibitors leading to recurrent mutations at hotspot positions within the NS3 protease sequence (Sorbo et al. 2018). Therefore, it was determined to what extent known NS3 RASs might disrupt the TCR 73-1 recognition motif (xxNGVxWxV). Interestingly, at position 54 of the NS3 protease the RASs T54A and T54S have been reported to frequently occur after DAA treatment (Qiu et al. 2009 and Tremeaux et al. 2016), which correspond to the amino acid positions P8 and P9 of the NS3 1073-1b epitope (Table 3). Furthermore, these mutations also occur after ribavirin treatment (Chevaliez et al. 2007) and appear even sporadically in treatment naive patients (Kuntzen et al. 2008). The RAS T54A, which creates the NS3 1073 epitope variants CVNGVCWAV and CINGVCWAV were both recognized by TCR 73-1 transduced PBMCs (Figure 7C,D). However, the RAS V55A/I leading to the NS3 1073-1b epitope variants CVNGVCWTA and CINGVCWTA is a mutation that disrupts recognition through TCR 73-1 (Figure 7). Finally, co-culturing TCR 73-1 transduced PBMCs with a panel of B-LCLs expressing different HLA molecules did not show any HLA-allo-reactivity except for minor reactivity to the cell line HOR expressing HLA-A*33:03:10, HLA-B*44:03:01 and HLA-Cw*14:03 (Figure 8A,B).

BMCs transduced with HCV-T003 were able to recognize T2 cells loaded with Core 132 peptide and responded with robust IFN-y secretion (938 pg/ml IFN-y) after re- stimulation with a concentration of up to 10-10 M peptide showing a functional avidity of EC50 of 2x10-8 M (Figure 9A,B). Alanine scan analysis of HCV-T003 revealed that amino acid positions 4,5,6,7 and 9 of the cognate Core 132 epitope (DLMGYIPLV) were minimally needed for antigen recognition revealing the recognition motif (xxxGYIPxV) for this TCR (Figure 9C). No peptide with the same recognition motif was found in the human proteome. However, different Core 132 epitope variants containing this recognition motif were found in a variety of HCV strains of genotypes 2a, 2b, 2c, 2k, 5a, 6a, 6b, 6d and 6k. Finally, co-culture of HCV-T003-transduced PBMC with a panel of B-LCLs expressing different HLA molecules revealed no HLA-allo-reactivity (Figure 8C,D).

PBMCs transduced with HCV-T004 responded with robust IFN-y secretion to co-culture with until 10-10 M stimulating peptide L5 (secreting 778 pg/ml IFN-y) and peptide M5 (secreting 1375 pg/ml IFN-y) (Figure 10A). The functional avidity of HCV-T004 for both peptides was found to be identical with an EC50 of 6.572x10⁻⁸ M for its cognate peptide epitope L5 and an EC50 = 6.551x10⁻⁸ M for the M5 peptide variant (Figure 10C). HCV-T005 transduced PBMCs responded with robust IFN-y secretion to co-culture with up until 10⁻¹¹ M stimulating M5 peptide (secreting 1183.63 pg/ml IFN-y) and L5 peptide (secreting 875.431 pg/ml IFN-y) (Figure 10B). However, the functional avidity of HCV-T005 for its cognate peptide epitope M5 was higher with an EC50 of 2.13x10⁻⁹ M than the functional avidity for the L5 peptide, which was 1.251x10⁻⁸ M (Figure 10D). Next, the individual L5/M5 epitope recognition patterns of both TCRs were further analyzed using an alanine scan panel based on epitopes L5 and M5. Since the cognate epitopes contain alanine at positions 4 and 9, these two positions were omitted in the analysis. Here, a drop in IFN-y secretion below 50% was set as a threshold for disruption of TCR recognition. It was found that recognition through HCV-T004 was disrupted by mutating epitope positions 1, 2, 5, 6 and 7 of both peptides from the L5 and the M5 panel, while alanine mutations at position 3 led to a drastic increase in recognition (Figure 10E). Overall, the effects to all distinct mutated positions were identical for the tested L5 and M5 peptide panel, suggesting that HCV-T004 uses a similar recognition pattern for recognizing L5/M5 peptides presented in HLA-A2 molecules (Figure 10E). However, the same panel used on HCV-T005-transduced PBMCs showed that this TCR uses a dissimilar pattern for L5 and M5 recognition. Here, alanine mutations at positions 1, 2 and 7 disrupted recognition throughout the L5/M5 panel, while mutating positions 3 and 5 led to a drastic increase in recognition (Figure 10G). Moreover, the recognition pattern for position 6, 8 and 10 were completely different for peptides from panel L5 and M5 (Figure 10G). Overall, this suggests that HCV-T004 recognizes L5/M5 peptides presented in HLA-A2 molecules through different recognition patterns. Taken together, HCV-T004 and HCV-T005 recognize their cognate peptide epitope and show cross- reactivity towards peptide M5 and L5. Interestingly, HCV-T005 recognizes its cognate epitope M5 with higher functional avidity than L5 and seems to achieve M5 recognition and L5 cross-recognition through distinct recognition patterns.

### Example 5: NS3 1406-1a-specific TCRs exhibit a biased TCR α/β chain and CDR3α region motif usage

Interestingly, HCV-T004 was found to harbor a TCRα chain (TRAV38-2/AJ30*1) that is identical to the TCRα chains of other NS31406-1a specific TCRs isolated from human settings. One study described a NS3 1406-1a (L5) specific T-cell clone that was identified from a HLA-A2 negative HCV-infected patient that had received a HLA-A2 positive liver transplant and had developed a CD8+ T-cell anti-graft response (Callender et al. 2006). The isolated allo-reactive TCR not only used the same TCRα chain as HCV-T004, but also contained a CDR3α region motif that was nearly identical to the one used by HCV-T004. However, the TCRβ chain of this allo-reactive TCR was different from the one used by HCV-T004 (Table 4). Another study aimed at establishing a novel enrichment and isolation technique for high affinity T-cell receptors from the blood of pathogen inexperienced human donors. Therein, NS3 1406-1a (L5)-specific CD8+ T-cell precursors were NS3 1406-1a (L5)-tetramer enriched from blood and sequenced through a single cell PCR technique revealing the TCRα and TCRβ chain combinations per sorted cell (Zhang et al.2016). Their data show that NS3 1406-1a (L5)-specific TCRs present in the naive T-cell precursor repertoire (of n=2 donors) also exhibit a strong bias towards TRAV38-2 TCR chain usage and even occur frequently in combination with the TCR Chain TRBV25-1*01. Also the CDR3α region of one described TCR was identical to the one from HCV-T004, while other TCRs used CDR3α regions that were at least highly similar to the CDR3α region of HCV-T004 (Table 4). Taken together these data indicate the existence of a public NS3 1406-1a (L5)-specific TCR in the human T-cell repertoire.

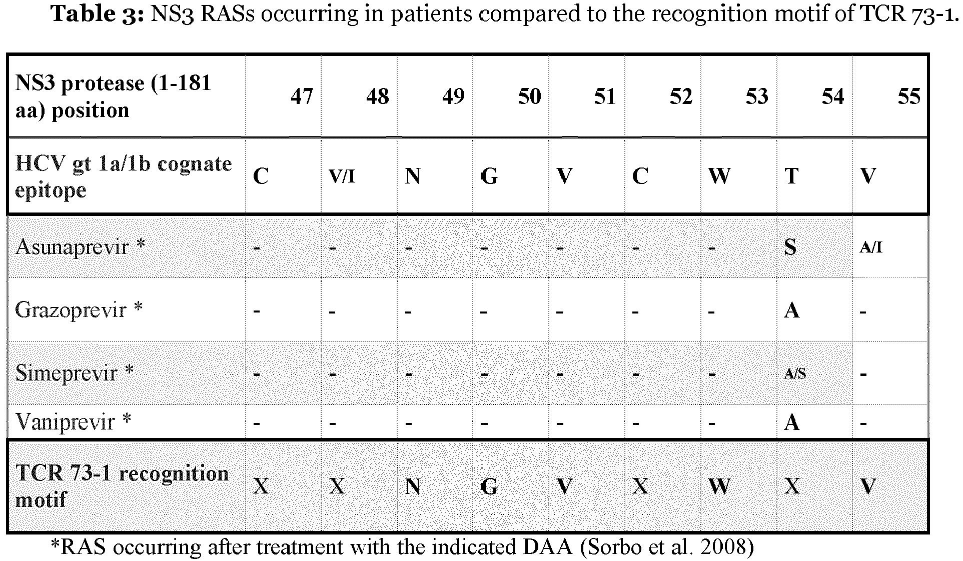

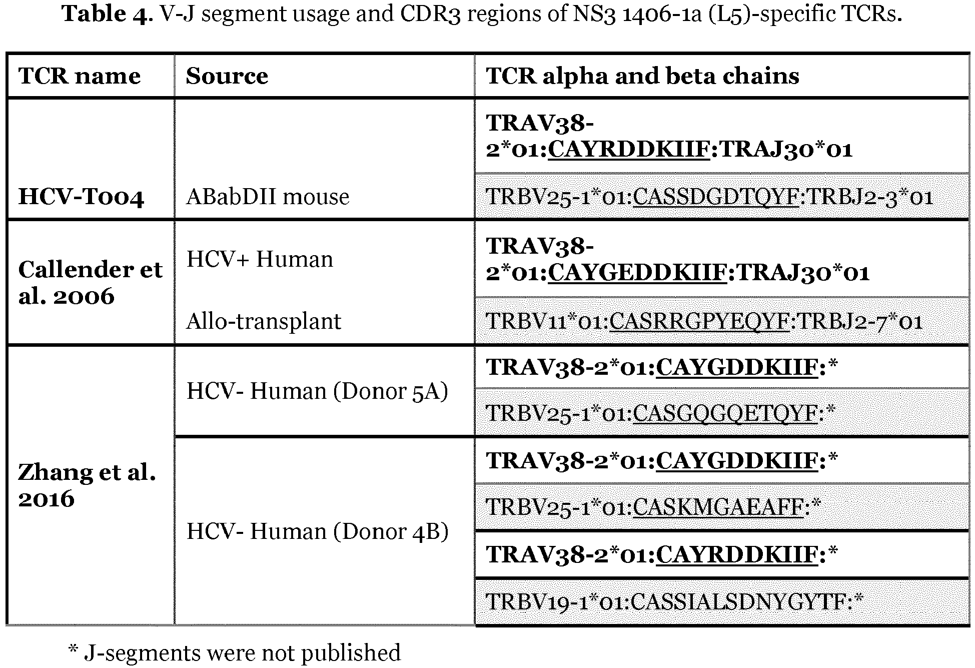

## Claims

1. **An isolated antigen binding protein (ABP) which specifically binds to a Hepatitis C Virus (HCV) associated antigenic peptide, or a variant thereof,** wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 3, 8, 13, 18, 23, 28, 33, 38, 43, and 48.

2. The isolated ABP of claim 1, wherein the ABP is an antibody, or an antigen binding derivative or fragment thereof, or, preferably, is a T cell receptor (TCR), or an antigen binding derivative or -fragment thereof.

3. The isolated ABP of claims 1 or 2, wherein said ABP binds to a human leucocyte antigen (HLA) presenting the antigenic peptide.

4. The isolated ABP of any one of claims 1 to 3, comprising a TCR α or γ chain; and/or a TCR β or δ chain; wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 3, 13, 23, 33, and 43; and/or wherein the TCR β or δ chain comprises a CDR₃ having an amino acid sequence with at least 80% sequence identity to, or having no more than three or two, preferably no more than one amino acid substitution(s), deletion(s) or insertion(s) compared to, a sequence selected from SEQ ID Nos: 8, 18, 28, 38, and 48.

5. The isolated ABP of claim 4, wherein the TCR α or γ chain further comprises a CDR1 having an amino acid sequence with at least 80% sequence identity to, or having no more than three or two, preferably no more than one amino acid substitution(s), deletion(s) or insertion(s) compared to, a sequence selected from SEQ ID Nos: 1, 11, 21, 31, and 41; and/or a CDR2 having an amino acid sequence with at least 80% sequence identity to, or having no more than three or two, preferably no more than one amino acid substitution(s), deletion(s) or insertion(s) compared to, a sequence selected from SEQ ID Nos: 2, 12, 22, 32, and 42.

6. The isolated ABP of claim 4 or 5, wherein the TCR β or δ chain further comprises a CDR1 having an amino acid sequence with at least 80% sequence identity to, or having no more than three or two, preferably no more than one amino acid substitution(s), deletion(s) or insertion(s) compared to, a sequence selected from SEQ ID Nos: 6, 16, 26, 36, and 46; and/or a CDR2 having an amino acid sequence with at least 80% sequence identity to, or having no more than three or two, preferably no more than one amino acid substitution(s), deletion(s) or insertion(s) compared to, a sequence selected from SEQ ID Nos: 7, 17, 27, 37, and 47.

7. The isolated ABP of any one of claims 1 to 6, wherein the ABP is a TCR, or a fragment thereof, composed of at least one TCR α and one TCR β chain sequence, wherein said TCR α chain sequence comprises the CDR1 to CDR3 sequences having the amino acid sequences of SEQ ID NO: 1 to 3, and said TCR β chain sequence comprises the CDR1 to CDR3 sequences having the amino acid sequences of SEQ ID NO: 6 to 8; or wherein said TCR α chain sequence comprises the CDR1 to CDR3 sequences having the amino acid sequences of SEQ ID NO: 11 to 13, and said TCR β chain sequence comprises the CDR1 to CDR3 sequences having the amino acid sequences of SEQ ID NO: 16 to 18; or wherein said TCR α chain sequence comprises the CDR1 to CDR3 sequences having the amino acid sequences of SEQ ID NO: 21 to 23, and said TCR β chain sequence comprises the CDR1 to CDR3 sequences having the amino acid sequences of SEQ ID NO: 26 to 28; or wherein said TCR α chain sequence comprises the CDR1 to CDR3 sequences having the amino acid sequences of SEQ ID NO: 31 to 33, and said TCR β chain sequence comprises the CDR1 to CDR3 sequences having the amino acid sequences of SEQ ID NO: 36 to 38; or wherein said TCR α chain sequence comprises the CDR1 to CDR3 sequences having the amino acid sequences of SEQ ID NO: 41 to 43, and said TCR β chain sequence comprises the CDR1 to CDR3 sequences having the amino acid sequences of SEQ ID NO: 46 to 48; each CDR independently having no more than three or two, preferably no more than one amino acid substitution(s), deletion(s) or insertion(s) compared to these sequences.

8. **An isolated nucleic acid** encoding for an ABP of any one of claims 1 to 7.

9. The isolated nucleic acid of claim 8, comprising a nucleic acid sequence having at least 80% sequence identity to, or having no more than 20, preferably no more than 15, more preferably no more than 10, 5, three or two, preferably no more than one nucleic acid substitution(s), deletion(s) or insertion(s) compared to, a nucleic acid sequence selected from SEQ ID Nos 5, 10, 15, 20, 25, 30, 35, 40, and 45.

10. **A recombinant vector** comprising a nucleic acid of claim 8 or 9.

11. **A recombinant host cell** comprising an ABP of any one of claims 1 to 7, or a nucleic acid of claim 8 or 9, or a vector of claim 10.

12. The recombinant host cell of claim 11, wherein the cell is a lymphocyte, preferably a T lymphocyte or T lymphocyte progenitor, more preferably a CD4 or a CD8 positive T-cell.

13. **A pharmaceutical composition** comprising the ABP of any one of claims 1 to 7, or a nucleic acid of claim 8 or 9, or a vector of claim 10, or the host cell of claim 11 or 12, and a pharmaceutical acceptable carrier, stabilizer and/or excipient; preferably, wherein the pharmaceutical composition comprises at least two different ABP of any one of claims 1 to 7.

14. **A compound or composition for use in medicine,** wherein the compound or composition is selected from the ABP of any one of claims 1 to 7, or a nucleic acid of claim 8 or 9, or a vector of claim 10, or the host cell of claim 11 or 12, or the pharmaceutical composition of claim 13.

15. The compound or composition for use of claim 14, wherein the use is for a treatment of an acute or chronic virus infection, preferably wherein the infection is an infection with a Hepatitis virus, such as HCV.

16. **A method of manufacturing a HCV specific antigen recognizing construct expressing cell line,** comprising
(i) providing a suitable host cell,
(ii) providing a genetic construct comprising a coding sequence encoding the ABP according to any of claims 1 to 7,
(iii) introducing into said suitable host cell said genetic construct,
(iv) expressing said genetic construct by said suitable host cell.

17. The method of claims 16, wherein said suitable host cell is a human T lymphocyte.


SEQUENCE LISTING

<110>  MAX-DELBRÜCK-CENTRUM FÜR MOLEKULARE MEDIZIN in der
       Helmholtz-Gemeinschaft
       Charité - Universitätsmedizin Berlin

<120>  T-CELL RECEPTORS SPECIFIC FOR HEPATITIS C VIRUS PEPTIDES

<130>  M35053EP

<160>  65

<170>  PatentIn version 3.5

<210>  1
<211>  7
<212>  PRT
<213>  Homo Sapiens

<400>  1

Thr Ser Asp Pro Ser Tyr Gly
1               5

<210>  2
<211>  8
<212>  PRT
<213>  Homo Sapiens

<400>  2

Gln Gly Ser Tyr Asp Gln Gln Asn
1               5

<210>  3
<211>  14
<212>  PRT
<213>  Homo Sapiens

<400>  3

Ala Met Arg Glu Tyr Ser Gly Gly Ser Asn Tyr Lys Leu Thr
1               5                   10

<210>  4
<211>  117
<212>  PRT
<213>  Homo Sapiens

<400>  4

Ala Gln Lys Ile Thr Gln Thr Gln Pro Gly Met Phe Val Gln Glu Lys
1               5                   10                  15

Glu Ala Val Thr Leu Asp Cys Thr Tyr Asp Thr Ser Asp Pro Ser Tyr
            20                  25                  30

Gly Leu Phe Trp Tyr Lys Gln Pro Ser Ser Gly Glu Met Ile Phe Leu

35                  40                  45

Ile Tyr Gln Gly Ser Tyr Asp Gln Gln Asn Ala Thr Glu Gly Arg Tyr
    50                  55                  60

Ser Leu Asn Phe Gln Lys Ala Arg Lys Ser Ala Asn Leu Val Ile Ser
65                  70                  75                  80

Ala Ser Gln Leu Gly Asp Ser Ala Met Tyr Phe Cys Ala Met Arg Glu
                85                  90                  95

Tyr Ser Gly Gly Ser Asn Tyr Lys Leu Thr Phe Gly Lys Gly Thr Leu
            100                 105                 110

Leu Thr Val Asn Pro
        115

<210>  5
<211>  352
<212>  DNA
<213>  Homo Sapiens

<400>  5
gcccagaaga tcacccagac ccagcccggc atgttcgtgc aggaaaaaga agccgtgacc      60

ctggactgca cctacgacac cagcgaccct agctacggac tgttttggta caagcagccc     120

agcagcggcg agatgatctt cctgatctac cagggctcct acgaccagca gaacgccacc     180

gagggccggt acagcctgaa cttccagaag gcccggaagt ccgccaacct cgtgatcagc     240

gctagccagc tgggcgacag cgccatgtac ttttgcgcca tgagagagta cagcggcggc     300

tccaactaca agctgacctt cggcaagggc acactgctga ccgtgaaccc ca             352

<210>  6
<211>  5
<212>  PRT
<213>  Homo Sapiens

<400>  6

Met Asp His Glu Asn
1               5

<210>  7
<211>  6
<212>  PRT
<213>  Homo Sapiens

<400>  7

Ser Tyr Asp Val Lys Met
1               5

<210>  8
<211>  14
<212>  PRT
<213>  Homo Sapiens

<400>  8

Ala Ser Ser Leu Leu Leu Ala Gly Val Gly Glu Thr Gln Tyr
1               5                   10

<210>  9
<211>  115
<212>  PRT
<213>  Homo Sapiens

<400>  9

Asp Val Lys Val Thr Gln Ser Ser Arg Tyr Leu Val Lys Arg Thr Gly
1               5                   10                  15

Glu Lys Val Phe Leu Glu Cys Val Gln Asp Met Asp His Glu Asn Met
            20                  25                  30

Phe Trp Tyr Arg Gln Asp Pro Gly Leu Gly Leu Arg Leu Ile Tyr Phe
        35                  40                  45

Ser Tyr Asp Val Lys Met Lys Glu Lys Gly Asp Ile Pro Glu Gly Tyr
    50                  55                  60

Ser Val Ser Arg Glu Lys Lys Glu Arg Phe Ser Leu Ile Leu Glu Ser
65                  70                  75                  80

Ala Ser Thr Asn Gln Thr Ser Met Tyr Leu Cys Ala Ser Ser Leu Leu
                85                  90                  95

Leu Ala Gly Val Gly Glu Thr Gln Tyr Phe Gly Pro Gly Thr Arg Leu
            100                 105                 110

Leu Val Leu
        115

<210>  10
<211>  346
<212>  DNA
<213>  Homo Sapiens

<400>  10
gacgtgaaag tgacccagag cagcagatac ctcgtgaagc ggaccggcga gaaggtgttc      60

ctggaatgcg tgcaggacat ggaccacgag aatatgttct ggtacagaca ggaccccggc     120

ctgggcctgc ggctgatcta cttcagctac gacgtgaaga tgaaggaaaa gggcgacatc     180

cccgagggct acagcgtgtc cagagagaag aaagagcggt tcagcctgat cctggaaagc     240

gccagcacca accagaccag catgtacctg tgcgccagct ccctgctgct ggctggcgtg     300

ggagagacac agtactttgg ccctggcacc agactgctgg tgctgg                    346

<210>  11
<211>  5
<212>  PRT
<213>  Homo Sapiens

<400>  11

Asp Ser Val Asn Asn
1               5

<210>  12
<211>  5
<212>  PRT
<213>  Homo Sapiens

<400>  12

Ile Pro Ser Gly Thr
1               5

<210>  13
<211>  12
<212>  PRT
<213>  Homo Sapiens

<400>  13

Ala Val Glu Pro Asn Ser Gly Asn Thr Pro Leu Val
1               5                   10

<210>  14
<211>  110
<212>  PRT
<213>  Homo Sapiens

<400>  14

Gly Ile Gln Val Glu Gln Ser Pro Pro Asp Leu Ile Leu Gln Glu Gly
1               5                   10                  15

Ala Asn Ser Thr Leu Arg Cys Asn Phe Ser Asp Ser Val Asn Asn Leu
            20                  25                  30

Gln Trp Phe His Gln Asn Pro Trp Gly Gln Leu Ile Asn Leu Phe Tyr
        35                  40                  45

Ile Pro Ser Gly Thr Lys Gln Asn Gly Arg Leu Ser Ala Thr Thr Val
    50                  55                  60

Ala Thr Glu Arg Tyr Ser Leu Leu Tyr Ile Ser Ser Ser Gln Thr Thr
65                  70                  75                  80

Asp Ser Gly Val Tyr Phe Cys Ala Val Glu Pro Asn Ser Gly Asn Thr
                85                  90                  95

Pro Leu Val Phe Gly Lys Gly Thr Arg Leu Ser Val Ile Ala
            100                 105                 110

<210>  15
<211>  331
<212>  DNA
<213>  Homo Sapiens

<400>  15
ggaatacaag tggagcagag tcctccagac ctgattctcc aggagggagc caattccacg      60

ctgcggtgca atttttctga ctctgtgaac aatttgcagt ggtttcatca aaacccttgg     120

ggacagctca tcaacctgtt ttacattccc tcagggacaa aacagaatgg aagattaagc     180

gccacgactg tcgctacgga acgctacagc ttattgtaca tttcctcttc ccagaccaca     240

gactcaggcg tttatttctg tgctgtggag ccgaattcag gaaacacacc tcttgtcttt     300

ggaaagggca caagactttc tgtgattgca a                                    331

<210>  16
<211>  5
<212>  PRT
<213>  Homo Sapiens

<400>  16

Met Asp His Glu Asn
1               5

<210>  17
<211>  6
<212>  PRT
<213>  Homo Sapiens

<400>  17

Ser Tyr Asp Val Lys Met
1               5

<210>  18
<211>  14
<212>  PRT
<213>  Homo Sapiens

<400>  18

Ala Ser Ser Leu Leu Leu Ala Gly Val Gly Glu Thr Gln Tyr
1               5                   10

<210>  19
<211>  115
<212>  PRT
<213>  Homo Sapiens

<400>  19

Asp Val Lys Val Thr Gln Ser Ser Arg Tyr Leu Val Lys Arg Thr Gly
1               5                   10                  15

Glu Lys Val Phe Leu Glu Cys Val Gln Asp Met Asp His Glu Asn Met
            20                  25                  30

Phe Trp Tyr Arg Gln Asp Pro Gly Leu Gly Leu Arg Leu Ile Tyr Phe
        35                  40                  45

Ser Tyr Asp Val Lys Met Lys Glu Lys Gly Asp Ile Pro Glu Gly Tyr
    50                  55                  60

Ser Val Ser Arg Glu Lys Lys Glu Arg Phe Ser Leu Ile Leu Glu Ser
65                  70                  75                  80

Ala Ser Thr Asn Gln Thr Ser Met Tyr Leu Cys Ala Ser Ser Leu Leu
                85                  90                  95

Leu Ala Gly Val Gly Glu Thr Gln Tyr Phe Gly Pro Gly Thr Arg Leu
            100                 105                 110

Leu Val Leu
        115

<210>  20
<211>  346
<212>  DNA
<213>  Homo Sapiens

<400>  20
gacgtgaaag tgacccagag cagcagatac ctcgtgaagc ggaccggcga gaaggtgttc      60

ctggaatgcg tgcaggacat ggaccacgag aatatgttct ggtacagaca ggaccccggc     120

ctgggcctgc ggctgatcta cttcagctac gacgtgaaga tgaaggaaaa gggcgacatc     180

cccgagggct acagcgtgtc cagagagaag aaagagcggt tcagcctgat cctggaaagc     240

gccagcacca accagaccag catgtacctg tgcgccagct ccctgctgct ggctggcgtg     300

ggagagacac agtactttgg ccctggcacc agactgctgg tgctgg                    346

<210>  21
<211>  6
<212>  PRT
<213>  Homo Sapiens

<400>  21

Thr Ser Trp Trp Ser Tyr
1               5


<210>  22
<211>  8
<212>  PRT
<213>  Homo Sapiens

<400>  22

Arg Gln Gly Ser Asp Glu Gln Asn
1               5


<210>  23
<211>  12
<212>  PRT
<213>  Homo Sapiens

<400>  23

Ala Leu Gly Glu Asn Ser Gly Tyr Ser Thr Leu Thr
1               5                   10


<210>  24
<211>  114
<212>  PRT
<213>  Homo Sapiens

<400>  24

Ala Gln Lys Val Thr Gln Ala Gln Ser Ser Val Ser Met Pro Val Arg
1               5                   10                  15

Lys Ala Val Thr Leu Asn Cys Leu Tyr Glu Thr Ser Trp Trp Ser Tyr
            20                  25                  30

Tyr Ile Phe Trp Tyr Lys Gln Leu Pro Ser Lys Glu Met Ile Phe Leu
        35                  40                  45

Ile Arg Gln Gly Ser Asp Glu Gln Asn Ala Lys Ser Gly Arg Tyr Ser
    50                  55                  60

Val Asn Phe Lys Lys Ala Ala Lys Ser Val Ala Leu Thr Ile Ser Ala
65                  70                  75                  80

Leu Gln Leu Glu Asp Ser Ala Lys Tyr Phe Cys Ala Leu Gly Glu Asn
                85                  90                  95

Ser Gly Tyr Ser Thr Leu Thr Phe Gly Lys Gly Thr Met Leu Leu Val
            100                 105                 110

Ser Pro

<210>  25
<211>  343
<212>  DNA
<213>  Homo Sapiens

<400>  25
gcccagaaag tgacacaggc ccagtcctcc gtgtccatgc ctgtgcggaa agccgtgacc      60

ctgaactgcc tgtacgaaac ctcctggtgg tcctactaca tcttctggta caagcagctg     120

cccagcaaag agatgatctt cctgatccgg cagggcagcg acgagcagaa tgccaagagc     180

ggccggtaca gcgtgaactt caagaaagcc gccaagtccg tggccctgac catctctgct     240

ctgcagctgg aagatagcgc caagtacttc tgtgccctgg gcgagaactc cggctactcc     300

accctgacct ttggcaaggg caccatgctg ctggtgtccc cca                       343

<210>  26
<211>  5
<212>  PRT
<213>  Homo Sapiens

<400>  26

Lys Gly His Asp Arg
1               5

<210>  27
<211>  6
<212>  PRT
<213>  Homo Sapiens

<400>  27

Ser Phe Asp Val Lys Asp
1               5

<210>  28
<211>  14
<212>  PRT
<213>  Homo Sapiens

<400>  28

Ala Thr Ser Asp Leu Gly Leu Ala Gly Tyr Asn Glu Gln Phe
1               5                   10

<210>  29
<211>  115
<212>  PRT
<213>  Homo Sapiens

<400>  29

Asp Ala Asp Val Thr Gln Thr Pro Arg Asn Arg Ile Thr Lys Thr Gly
1               5                   10                  15

Lys Arg Ile Met Leu Glu Cys Ser Gln Thr Lys Gly His Asp Arg Met
            20                  25                  30

Tyr Trp Tyr Arg Gln Asp Pro Gly Leu Gly Leu Arg Leu Ile Tyr Tyr
        35                  40                  45

Ser Phe Asp Val Lys Asp Ile Asn Lys Gly Glu Ile Ser Asp Gly Tyr
    50                  55                  60

Ser Val Ser Arg Gln Ala Gln Ala Lys Phe Ser Leu Ser Leu Glu Ser
65                  70                  75                  80

Ala Ile Pro Asn Gln Thr Ala Leu Tyr Phe Cys Ala Thr Ser Asp Leu
                85                  90                  95

Gly Leu Ala Gly Tyr Asn Glu Gln Phe Phe Gly Pro Gly Thr Arg Leu
            100                 105                 110

Thr Val Leu
        115

<210>  30
<211>  346
<212>  DNA
<213>  Homo Sapiens

<400>  30
gacgccgatg tgacccagac cccccggaac agaatcacca agaccggcaa gcggatcatg      60

ctggaatgca gccagaccaa gggccacgac cggatgtact ggtacagaca ggaccctggc     120

ctgggcctgc ggctgatcta ctacagcttc gacgtgaagg acatcaacaa gggcgagatc     180

agcgacggct acagcgtgtc cagacaggct caggccaagt tcagcctgtc cctggaaagc     240

gccatcccca accagaccgc cctgtacttc tgcgccacaa gcgatctggg cctggccggc     300

tacaacgagc agttctttgg ccctggcacc cggctgaccg tgctgg                    346

<210>  31
<211>  7
<212>  PRT
<213>  Homo Sapiens

<400>  31

Thr Ser Glu Ser Asp Tyr Tyr
1               5

<210>  32
<211>  8
<212>  PRT
<213>  Homo Sapiens

<400>  32

Gln Glu Ala Tyr Lys Gln Gln Asn
1               5


<210>  33
<211>  8
<212>  PRT
<213>  Homo Sapiens

<400>  33

Ala Tyr Arg Asp Asp Lys Ile Ile
1               5


<210>  34
<211>  111
<212>  PRT
<213>  Homo Sapiens

<400>  34

Ala Gln Thr Val Thr Gln Ser Gln Pro Glu Met Ser Val Gln Glu Ala
1               5                   10                  15

Glu Thr Val Thr Leu Ser Cys Thr Tyr Asp Thr Ser Glu Ser Asp Tyr
            20                  25                  30

Tyr Leu Phe Trp Tyr Lys Gln Pro Pro Ser Arg Gln Met Ile Leu Val
        35                  40                  45

Ile Arg Gln Glu Ala Tyr Lys Gln Gln Asn Ala Thr Glu Asn Arg Phe
    50                  55                  60

Ser Val Asn Phe Gln Lys Ala Ala Lys Ser Phe Ser Leu Lys Ile Ser
65                  70                  75                  80

Asp Ser Gln Leu Gly Asp Ala Ala Met Tyr Phe Cys Ala Tyr Arg Asp
                85                  90                  95

Asp Lys Ile Ile Phe Gly Lys Gly Thr Arg Leu His Ile Leu Pro
            100                 105                 110


<210>  35
<211>  334
<212>  DNA
<213>  Homo Sapiens

<400>  35

gcccagaccg tgacacagag ccagcccgag atgagcgtgc aggaagccga gacagtgacc 60

ctgagctgca cctacgacac cagcgagtcc gactactacc tgttctggta caagcagccc 120

cccagccggc agatgatcct cgtgatcaga caggaagcct acaagcagca gaacgccacc 180

gagaacagat tcagcgtgaa cttccagaag gccgccaaga gctttagcct gaagatcagc 240

gacagccagc tgggcgacgc cgccatgtac ttttgcgcct accgggacga taagatcatc 300

ttcggcaagg gcacccggct gcacatcctg ccca 334

<210> 36
<211> 5
<212> PRT
<213> Homo Sapiens

<400> 36

Met Gly His Asp Lys
1               5

<210> 37
<211> 6
<212> PRT
<213> Homo Sapiens

<400> 37

Ser Tyr Gly Val Asn Ser
1               5

<210> 38
<211> 9
<212> PRT
<213> Homo Sapiens

<400> 38

Ala Ser Ser Asp Gly Asp Thr Gln Tyr
1               5

<210> 39
<211> 110
<212> PRT
<213> Homo Sapiens

<400> 39

Glu Ala Asp Ile Tyr Gln Thr Pro Arg Tyr Leu Val Ile Gly Thr Gly
1               5                   10                  15

Lys Lys Ile Thr Leu Glu Cys Ser Gln Thr Met Gly His Asp Lys Met
            20                  25                  30

Tyr Trp Tyr Gln Gln Asp Pro Gly Met Glu Leu His Leu Ile His Tyr
        35                  40                  45

Ser Tyr Gly Val Asn Ser Thr Glu Lys Gly Asp Leu Ser Ser Glu Ser
    50                  55                  60

Thr Val Ser Arg Ile Arg Thr Glu His Phe Pro Leu Thr Leu Glu Ser
65                  70                  75                  80

Ala Arg Pro Ser His Thr Ser Gln Tyr Leu Cys Ala Ser Ser Asp Gly
                85                  90                  95

Asp Thr Gln Tyr Phe Gly Pro Gly Thr Arg Leu Thr Val Leu
            100                 105                 110

<210>  40
<211>  331
<212>  DNA
<213>  Homo Sapiens

<400>  40
gaagccgaca tctaccagac ccccagatac ctcgtgatcg gcaccggcaa gaaaatcacc      60

ctggaatgca gccagaccat gggccacgac aagatgtact ggtatcagca ggaccccggc     120

atggaactgc acctgatcca ctacagctac ggcgtgaaca gcaccgagaa gggcgacctg     180

agcagcgaga gcaccgtgtc cagaatccgg accgagcact tcccactgac cctggaaagc     240

gctagaccca gccacaccag ccagtacctg tgtgccagca gcgacggcga cacccagtat     300

tttggccctg gcaccagact gaccgtgctg g                                    331

<210>  41
<211>  7
<212>  PRT
<213>  Homo Sapiens

<400>  41

Thr Ser Glu Ser Asp Tyr Tyr
1               5

<210>  42
<211>  8
<212>  PRT
<213>  Homo Sapiens

<400>  42

Gln Glu Ala Tyr Lys Gln Gln Asn
1               5

<210>  43
<211>  10
<212>  PRT
<213>  Homo Sapiens

<400>  43

Ala Tyr Ser Glu Tyr Gly Asn Lys Leu Val
1               5                   10


<210>  44
<211>  113
<212>  PRT
<213>  Homo Sapiens

<400>  44

Ala Gln Thr Val Thr Gln Ser Gln Pro Glu Met Ser Val Gln Glu Ala
1               5                   10                  15

Glu Thr Val Thr Leu Ser Cys Thr Tyr Asp Thr Ser Glu Ser Asp Tyr
            20                  25                  30

Tyr Leu Phe Trp Tyr Lys Gln Pro Pro Ser Arg Gln Met Ile Leu Val
        35                  40                  45

Ile Arg Gln Glu Ala Tyr Lys Gln Gln Asn Ala Thr Glu Asn Arg Phe
    50                  55                  60

Ser Val Asn Phe Gln Lys Ala Ala Lys Ser Phe Ser Leu Lys Ile Ser
65                  70                  75                  80

Asp Ser Gln Leu Gly Asp Ala Ala Met Tyr Phe Cys Ala Tyr Ser Glu
                85                  90                  95

Tyr Gly Asn Lys Leu Val Phe Gly Ala Gly Thr Ile Leu Arg Val Lys
            100                 105                 110

Ser


<210>  45
<211>  340
<212>  DNA
<213>  Homo Sapiens

<400>  45
gcccagaccg tgactcagag ccagcccgag atgagcgtgc aggaagccga gacagtgacc      60

ctgagctgca cctacgacac cagcgagagc gactactacc tgttctggta caagcagccc     120

cccagccggc agatgatcct cgtgattaga caggaagcct ataagcagca gaacgccaca     180

gagaacagat tcagcgtgaa cttccagaag gccgccaaga gctttagcct gaagatcagc     240

gacagccagc tgggcgacgc cgccatgtat ttctgcgcct acagcgagta cggcaacaag     300

ctggtgttcg gagccggcac aatcctgaga gtgaagtcct                           340

<210>  46
<211>  5
<212>  PRT
<213>  Homo Sapiens

<400>  46

Met Asn His Glu Tyr
1               5


<210>  47
<211>  6
<212>  PRT
<213>  Homo Sapiens

<400>  47

Ser Met Asn Val Glu Val
1               5


<210>  48
<211>  13
<212>  PRT
<213>  Homo Sapiens

<400>  48

Ala Ser Ser Leu Phe Arg Ser Ser Tyr Asn Glu Gln Phe
1               5                   10


<210>  49
<211>  114
<212>  PRT
<213>  Homo Sapiens

<400>  49

Glu Ala Gln Val Thr Gln Asn Pro Arg Tyr Leu Ile Thr Val Thr Gly
1               5                   10                  15

Lys Lys Leu Thr Val Thr Cys Ser Gln Asn Met Asn His Glu Tyr Met
            20                  25                  30

Ser Trp Tyr Arg Gln Asp Pro Gly Leu Gly Leu Arg Gln Ile Tyr Tyr
        35                  40                  45

Ser Met Asn Val Glu Val Thr Asp Lys Gly Asp Val Pro Glu Gly Tyr
    50                  55                  60

Lys Val Ser Arg Lys Glu Lys Arg Asn Phe Pro Leu Ile Leu Glu Ser
65                  70                  75                  80

Pro Ser Pro Asn Gln Thr Ser Leu Tyr Phe Cys Ala Ser Ser Leu Phe

85                  90                  95

Arg Ser Ser Tyr Asn Glu Gln Phe Phe Gly Pro Gly Thr Arg Leu Thr
            100                 105                 110

Val Leu

<210>  50
<211>  343
<212>  DNA
<213>  Homo Sapiens

<400>  50
gaagcccaag tgacccagaa ccccagatac ctgatcaccg tgaccggcaa gaaactgacc      60

gtgacctgca gccagaacat gaaccacgag tacatgagct ggtacagaca ggaccccggc     120

ctgggcctgc ggcagatcta ctacagcatg aacgtggaag tgaccgacaa gggcgacgtg     180

cccgagggct acaaggtgtc ccggaaagag aagcggaact tcccactgat cctggaaagc     240

cccagcccca accagaccag cctgtacttc tgtgccagca gcctgttcag aagcagctac     300

aacgagcagt tcttcggccc tggcacccgg ctgaccgtgc tgg                       343

<210>  51
<211>  10
<212>  PRT
<213>  Hepacivirus C

<400>  51

Tyr Leu Leu Pro Arg Arg Gly Pro Arg Leu
1               5                   10

<210>  52
<211>  9
<212>  PRT
<213>  Hepacivirus C

<400>  52

Asp Leu Met Gly Tyr Ile Pro Leu Val
1               5

<210>  53
<211>  9
<212>  PRT
<213>  Hepacivirus C

<400>  53

Cys Val Asn Gly Val Cys Trp Thr Val
1               5

<210>  54
<211>  10
<212>  PRT
<213>  Hepacivirus C

<400>  54

Lys Leu Thr Gly Leu Gly Leu Asn Ala Val
1               5                   10


<210>  55
<211>  10
<212>  PRT
<213>  Hepacivirus C

<400>  55

Lys Leu Val Ala Leu Gly Ile Asn Ala Val
1               5                   10


<210>  56
<211>  10
<212>  PRT
<213>  Hepacivirus C

<400>  56

Lys Leu Val Ala Met Gly Ile Asn Ala Val
1               5                   10


<210>  57
<211>  575
<212>  PRT
<213>  Hepacivirus C

<400>  57

Met Ala Pro Ile Thr Ala Tyr Ser Gln Gln Thr Arg Gly Leu Leu Gly
1               5                   10                  15

Cys Ile Ile Thr Ser Leu Thr Gly Arg Asp Lys Asn Gln Val Asp Gly
            20                  25                  30

Glu Val Gln Val Leu Ser Thr Ala Thr Gln Ser Phe Leu Ala Thr Cys
        35                  40                  45

Val Asn Gly Val Cys Trp Thr Val Tyr His Gly Ala Gly Ser Lys Thr
    50                  55                  60

Leu Ala Gly Pro Lys Gly Pro Ile Thr Gln Met Tyr Thr Asn Val Asp
65                  70                  75                  80

Gln Asp Leu Val Gly Trp Pro Ala Pro Pro Gly Ala Arg Ser Met Thr
                85                  90                  95

Pro Cys Thr Cys Gly Ser Ser Asp Leu Tyr Leu Val Thr Arg His Ala
            100                 105                 110

Asp Val Ile Pro Val Arg Arg Arg Gly Asp Ser Arg Gly Ser Leu Leu
        115                 120                 125

Ser Pro Arg Pro Val Ser Tyr Leu Lys Gly Ser Ser Gly Gly Pro Leu
    130                 135                 140

Leu Cys Pro Ser Gly His Val Val Gly Ile Phe Arg Ala Ala Val Cys
145                 150                 155                 160

Thr Arg Gly Val Ala Lys Ala Val Asp Phe Ile Pro Val Glu Ser Met
                165                 170                 175

Glu Thr Thr Met Arg Ser Pro Val Phe Thr Asp Asn Ser Ser Pro Pro
            180                 185                 190

Ala Val Pro Gln Thr Phe Gln Val Ala His Leu His Ala Pro Thr Gly
        195                 200                 205

Ser Gly Lys Ser Thr Lys Val Pro Ala Ala Tyr Ala Ala Gln Gly Tyr
    210                 215                 220

Lys Val Leu Val Leu Asn Pro Ser Val Ala Ala Thr Leu Gly Phe Gly
225                 230                 235                 240

Ala Tyr Met Ser Lys Ala His Gly Ile Glu Pro Asn Ile Arg Thr Gly
                245                 250                 255

Val Arg Thr Ile Thr Thr Gly Gly Pro Ile Thr Tyr Ser Thr Tyr Gly
            260                 265                 270

Lys Phe Leu Ala Asp Gly Gly Cys Ser Gly Gly Ala Tyr Asp Ile Ile
        275                 280                 285

Ile Cys Asp Glu Cys His Ser Thr Asp Trp Thr Thr Ile Leu Gly Ile
    290                 295                 300

Gly Thr Val Leu Asp Gln Ala Glu Thr Ala Gly Ala Arg Leu Val Val
305                 310                 315                 320

Leu Ala Thr Ala Thr Pro Pro Gly Ser Ile Thr Val Pro His Pro Asn
                325                 330                 335

Ile Glu Glu Val Ala Leu Ser Asn Thr Gly Glu Ile Pro Phe Tyr Gly
            340                 345                 350

Lys Ala Ile Pro Ile Glu Ala Ile Lys Gly Gly Arg His Leu Ile Phe
        355                 360                 365

Cys His Ser Lys Lys Lys Cys Asp Glu Leu Ala Ala Lys Leu Thr Gly
    370                 375                 380

Leu Gly Leu Asn Ala Val Ala Tyr Tyr Arg Gly Leu Asp Val Ser Val
385                 390                 395                 400

Ile Pro Thr Ser Gly Asp Val Val Val Val Ala Thr Asp Ala Leu Met
                405                 410                 415

Thr Gly Phe Thr Gly Asp Phe Asp Ser Val Ile Asp Cys Asn Thr Cys
            420                 425                 430

Val Thr Gln Thr Val Asp Phe Ser Leu Asp Pro Thr Phe Thr Ile Glu
        435                 440                 445

Thr Thr Thr Val Pro Gln Asp Ala Val Ser Arg Ser Gln Arg Arg Gly
    450                 455                 460

Arg Thr Gly Arg Gly Arg Ser Gly Ile Tyr Arg Phe Val Thr Pro Gly
465                 470                 475                 480

Glu Arg Pro Ser Gly Met Phe Asp Ser Ser Val Leu Cys Glu Cys Tyr
                485                 490                 495

Asp Ala Gly Cys Ala Trp Tyr Glu Leu Thr Pro Ala Glu Thr Thr Val
            500                 505                 510

Arg Leu Arg Ala Tyr Leu Asn Thr Pro Gly Leu Pro Val Cys Gln Asp
        515                 520                 525

His Leu Glu Phe Trp Glu Ser Val Phe Thr Gly Leu Thr His Ile Asp
    530                 535                 540

Ala His Phe Leu Ser Gln Thr Lys Gln Ala Gly Asp Asn Phe Pro Tyr
545                 550                 555                 560

Leu Val Ala Tyr Gln Ala Thr Val Cys Ala Arg Ala Gln Ala Pro
                565                 570                 575

<210>  58
<211>  191
<212>  PRT
<213>  Hepacivirus C

<400>  58

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Thr Trp Ala Gln Pro Gly
65                  70                  75                  80

Tyr Pro Trp Pro Leu Tyr Gly Asn Glu Gly Met Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala
            180                 185                 190


<210>  59
<211>  9
<212>  PRT
<213>  Hepacivirus C


<220>
<221>  VARIANT
<222>  (1)..(3)
<223>  Xaa can be any naturally occurring amino acid

<220>
<221>  VARIANT

<222>  (8)..(8)
<223>  Xaa can be any naturally occurring amino acid

<400>  59

Xaa Xaa Xaa Gly Tyr Ile Pro Xaa Val
1               5


<210>  60
<211>  11
<212>  PRT
<213>  Homo Sapiens

<400>  60

Cys Ala Tyr Gly Glu Asp Asp Lys Ile Ile Phe
1               5                   10


<210>  61
<211>  12
<212>  PRT
<213>  Homo Sapiens

<400>  61

Cys Ala Ser Arg Arg Gly Pro Tyr Glu Gln Tyr Phe
1               5                   10


<210>  62
<211>  10
<212>  PRT
<213>  Homo Sapiens

<400>  62

Cys Ala Tyr Gly Asp Asp Lys Ile Ile Phe
1               5                   10


<210>  63
<211>  11
<212>  PRT
<213>  Homo Sapiens

<400>  63

Cys Ala Ser Lys Met Gly Ala Glu Ala Phe Phe
1               5                   10


<210>  64
<211>  10
<212>  PRT
<213>  Homo Sapiens

<400>  64

Cys Ala Tyr Arg Asp Asp Lys Ile Ile Phe
1               5                   10

<210>  65
<211>  15
<212>  PRT
<213>  Homo Sapiens

<400>  65

Cys Ala Ser Ser Ile Ala Leu Ser Asp Asn Tyr Gly Tyr Thr Phe
1               5                   10                  15